(19)

Europäisches
Patentamt
European
Patent Office
Office européen
des brevets

(11) **EP 2 356 181 B1**

(12) **EUROPÄISCHE PATENTSCHRIFT**

(45) Veröffentlichungstag und Bekanntmachung des
Hinweises auf die Patenterteilung:
**24.07.2013 Patentblatt 2013/30**

(21) Anmeldenummer: **10742439.2**

(22) Anmeldetag: **09.08.2010**

(51) Int Cl.:
***C09C 1/00*** *(2006.01)*

(86) Internationale Anmeldenummer:
**PCT/EP2010/004865**

(87) Internationale Veröffentlichungsnummer:
**WO 2011/020570 (24.02.2011 Gazette 2011/08)**

(54) **HOCHGLÄNZENDE MEHRSCHICHTPERLGLANZPIGMENTE MIT FARBIGER INTERFERENZFARBE UND ENGER GRÖSSENVERTEILUNG UND VERFAHREN ZU DEREN HERSTELLUNG**

HIGH-GLOSS MULTILAYER EFFECT PIGMENTS HAVING A CHROMATIC INTERFERENCE COLOR AND A NARROW SIZE DISTRIBUTION, AND METHOD FOR THE PRODUCTION THEREOF

PIGMENTS NACRÉS MULTICOUCHE TRÈS BRILLANTS DOTÉS D'UNE TEINTE DE POLARISATION ET D'UNE ÉTROITE DISTRIBUTION GRANULOMÉTRIQUE ET PROCÉDÉ DE PRODUCTION

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **19.08.2009 DE 102009037934**

(43) Veröffentlichungstag der Anmeldung:
**17.08.2011 Patentblatt 2011/33**

(73) Patentinhaber: **Eckart GmbH**
**91235 Hartenstein (DE)**

(72) Erfinder:
• **SCHUMACHER, Dirk**
**91257 Pegnitz (DE)**

• **GRÜNER, Michael**
**91275 Auerbach (DE)**
• **KAUPP, Günter**
**91284 Neuhaus (DE)**

(74) Vertreter: **Walcher, Armin**
**Louis, Pöhlau, Lohrentz**
**Postfach 3055**
**90014 Nürnberg (DE)**

(56) Entgegenhaltungen:
**WO-A1-2009/103322 WO-A2-03/006558**
**WO-A2-2006/110359 US-A1- 2006 042 509**

## Beschreibung

Die vorliegende Erfindung betrifft hochglänzende Mehrschichtperlglanzpigmente mit farbiger Interferenzfarbe sowie ein Verfahren zur Herstellung derselben als auch deren Verwendung in kosmetischen Formulierungen, Kunststoffen, Folien, Textilien, keramischen Materialien, Gläsern, Beschichtungszusammensetzungen wie Farben, Druckfarben, Tinten, Lacken oder Pulverlacken.

Die optische Wirkung von Effektpigmenten beruht auf der gerichteten Reflexion von Licht an überwiegend flächig ausgebildeten, zueinander im Wesentlichen parallel ausgerichteten lichtbrechenden Pigmentteilchen. Diese Pigmentteilchen weisen in der Regel ein im Wesentlichen transparentes Substrat und eine oder mehrere Beschichtungen auf dem Substrat auf. Je nach Zusammensetzung der Beschichtung(en) der Pigmentteilchen erzeugen Interferenz-, Reflexions- und/ oder Absorptionsphänomene Farb- und Helligkeitseindrücke.

Unregelmäßigkeiten in der zu beschichtenden Substratoberfläche oder farbige Verunreinigungen des Substrats können zu unerwünschten Streulichteffekten und mithin zu einem verringerten Glanz sowie Farbunreinheiten im Endprodukt führen. Insbesondere bei Verwendung natürlicher Substratmaterialien, wie natürlichem Glimmer, treten diese unerwünschten Streulichteffekte und/ oder farblichen Verunreinigungen auf.

Der grundsätzliche Aufbau von Perlglanzpigmenten und mithin auch von Mehrschichtperlglanzpigmenten mit einer alternierenden Anordnung von hoch- und niedrigbrechenden Schichten ist bekannt. Exemplarisch wird auf die Patentanmeldungen DE 41 41 069 A1, DE 43 19 669 A1, DE 196 14 637 A1, DE 198 02 234 A1, DE 198 08 657 A1, DE 198 22 046 A1, DE 199 07 313 A1, DE 199 41 253 A1, DE 199 53 655 A1 oder DE 102 21 497 A1 verwiesen.

In der WO 2004/055119 A1 werden Interferenzpigmente auf der Basis von beschichteten plättchenförmigen Substraten beschrieben. Die Substrate sind hierbei mit einer ersten Schicht aus $SiO_2$ belegt, auf die anschließend eine hochbrechende Schicht, bestehend aus beispielsweise $TiO_2$, $ZrO_2$, $SnO_2$, $Cr_2O_3$, $Fe_2O_3$ oder $Fe_3O_4$ bzw. ein Interferenzsystem aus alternierenden hoch- und niedrigbrechenden Schichten aufgebracht ist. Optional können die Pigmente noch eine äußere Schutzschicht aufweisen. Auf diese Weise werden silberweiße Interferenzpigmente bzw. Interferenzpigmente mit brillanten Interferenzfarben erhalten, die sich durch anwendungstechnische Eigenschaften, wie mechanische Stabilität und Photostabilität auszeichnen, allerdings keinen hohen Glanz aufweisen. Die Interferenzpigmente zeigen keine oder nur eine geringe Winkelabhängigkeit der Farbe.

Thermisch und mechanisch stabile metalloxidbeschichtete Effektpigmente basierend auf dünnen Glasflakes mit einer Dicke s 1,0 μm sind aus der WO 2002/090448 A2 bekannt. Die Effektpigmente können mit einer oder mehreren hoch- und/ oder niedrigbrechenden Schicht(en) belegt sein. Die Glasflakes besitzen eine Erweichungstemperatur von ≥ 800°C.

Goniochromatische Glanzpigmente werden in EP 0 753 545 B2 beschrieben. Mindestens ein Schichtpaket aus einer farblosen niedrigbrechenden und einer reflektierenden, selektiv oder nichtselektiv absorbierenden Beschichtung sowie optional einer äußeren Schutzschicht wird hier auf ein mehrfach beschichtetes, hochbrechendes, nichtmetallisches, plättchenförmiges Substrat aufgebracht. Die Schichtdicke der farblosen niedrigbrechenden Beschichtung verringert sich bei steigender Anzahl der auf dem Substrat aufgebrachten Schichtpakete. Die goniochromatischen Glanzpigmente zeigen einen winkelabhängigen Farbwechsel zwischen mehreren intensiven Interferenzfarben.

Gemäß WO 2004/067645 A2 wird ein transparentes Substrat mit einer ungeraden Anzahl, mindestens drei, abwechselnd hoch- und niedrigbrechenden Schichten beschichtet. Der Brechungsindexunterschied der aneinandergrenzenden Schichten liegt bei mindestens 0,2. Mindestens eine der Schichten unterscheidet sich in ihrer optischen Dicke von den anderen. Somit besitzen die resultierenden Mehrschichteffektpigmente keinen Schichtaufbau, bei dem die optische Dicke jeder Schicht ein ungeradzahliges Vielfaches eines Viertels der zu interferierenden Lichtwellenlänge beträgt (kein "quarter-wave-stack"-Aufbau).

Mehrschichtige Interferenzpigmente mit kräftigen Interferenzfarben und/ oder einer starken Winkelabhängigkeit der Interferenzfarben, bestehend aus einem transparenten Trägermaterial, das mit alternierenden Schichten von Metalloxiden mit niedriger und hoher Brechzahl beschichtet ist, werden in EP 0 948 572 B1 beschrieben. Die Differenz der Brechzahlen beträgt mindestens 0,1. Die Anzahl und Dicke der Schichten ist abhängig vom gewünschten Effekt und dem verwendeten Substrat. Betrachtet man den Aufbau $TiO_2$-$SiO_2$-TiO2 auf einem Glimmersubstrat, so lassen sich bei Verwendung optisch dünner $TiO_2$- und $SiO_2$-Schichten mit einer Schichtdicke < 100 nm beispielsweise Pigmente mit blauer Interferenzfarbe erhalten, die farbkräftiger als reine $TiO_2$-Glimmerpigmente sind. Durch die Auffällung dicker $SiO_2$-Schichten mit einer Schichtdicke > 100 nm werden Pigmente mit einer stark ausgeprägten Winkelabhängigkeit der Interferenzfarbe erhalten.

Die optischen Eigenschaften von Effektpigmenten können nach der WO 2006/110359 A2 durch eine geeignete Partikelgrößenverteilung beeinflusst werden. Die hier beschriebenen klassierten mit einer einzigen Metalloxidschicht beschichteten Glasflakes weisen einen $D_{10}$ von wenigstens 9,5 μm, vorzugsweise von 9,5 μm, auf. Nachteilig ist, dass die Pigmente einen Größenbereich mit einem $D_{90}$-Wert von maximal 85 μm, vorzugsweise von etwa 45 μm, aufzuweisen haben.

Die US 2006/0042509 A1 offenbart Perlglanzpigmente mit einem Metalloxid-haltigen plättchenförmigen Substrat und einer ersten und zweiten Schutzschicht, wobei die erste Schutzschicht Ceroxid und/oder Cerhydroxid umfasst und eine zweite Schutzschicht aus $SiO_2$, auf die eine organisch-chemische Nachbeschichtung aufgebracht ist.

WO 2009/103322 A1 betrifft Effektpigmente, umfassend künstliche plättchenförmige Substrate, die mindestens eine optisch wirksame Beschichtung aufweisen, wobei die Effektpigmente eine Volumen-gemittelte Größensummendurchgangsverteilungskurve mit den Kennzahlen $D_{10}$, $D_{50}$ und $D_{90}$ aufweisen, wobei diese Größensummendurchgangsverteilungskurve einen Span $\Delta D$ von 0,7 - 1,4 hat. Der Span $\Delta D$ berechnet sich gemäß der folgenden Formel: $\Delta D = (D_{90} - D_{10}) / D_{50}$. Die mittlere Dicke der künstlichen plättchenförmigen Substrate liegt in einem Bereich von 500 bis 200 nm.

Die WO 03/006558 A2 offenbart Mehrschichteffektpigmente auf Glasplättchen, wobei die Glasplättchen mit alternierenden Schichten mit hohem und niedrigem Brechungsindex beschichtet sind und die Glasplättchen wenigstens drei Schichten aufweisen.

Im Stand der Technik sind diverse mehrschichtige Perlglanzpigmente offenbart, die über ansprechende optische Eigenschaften verfügen. Dennoch besteht weiterhin ein Bedarf an verbesserten Produkten.

Aufgabe der vorliegenden Erfindung ist es, mehrschichtige Perlglanzpigmente mit verbessertem Glanz bereit zu stellen. Die mehrschichtigen Perlglanzpigmente sollten weiterhin ein höheres Chroma aufweisen.

Mehrschichtige Perlglanzpigmente mit einem Farbflop sollten möglichst einen höheren Farbflop als die mehrschichtigen Perlglanzpigmente aus dem Stand der Technik aufweisen.

Die Aufgabe wurde gelöst durch Bereitstellung von Mehrschichtperlglanzpigmenten, umfassend mit optisch wirksamer Beschichtung versehene plättchenförmige transparente Substrate, wobei die optisch wirksame Beschichtung wenigstens

a) eine absorbierende hochbrechende Schicht A mit einem Brechungsindex $n \geq 1,8$,
b) eine niedrigbrechende Schicht B mit einem Brechungsindex $n < 1,8$,
c) eine hochbrechende Schicht C mit einem Brechungsindex $n \geq 1,8$
sowie
d) optional wenigstens eine äußere Schutzschicht D

umfasst und wobei die Mehrschichtperlglanzpigmente eine Summenhäufigkeitsverteilung einer volumengemittelten Größenverteilungsfunktion mit den Kennzahlen $D_{10}$, $D_{50}$, $D_{90}$ und einem Span $\Delta D$ aus einem Bereich von 0,7 - 1,4 aufweisen, wobei der Span $\Delta D$ gemäß Formel (I)

$$\Delta D = (D_{90} - D_{10}) / D_{50} \qquad\qquad (I)$$

berechnet ist.

Bevorzugte Weiterbildungen sind in den abhängigen Ansprüchen 2 bis 10 angegeben.

Die Aufgabe wurde weiterhin gelöst durch Bereitstellung eines Verfahrens zum Herstellen der erfindungsgemäßen Mehrschichtperlglanzpigmente, wobei das Verfahren folgende Schritte umfasst:

(i) Größenklassieren der zu beschichtenden plättchenförmigen transparenten Substrate, so dass die zu beschichtenden plättchenförmigen transparenten Substrate eine volumengemittelte Größenverteilungsfunktion mit den Kennzahlen $D_{10}$, $D_{50}$, $D_{90}$ und einem Span $\Delta D$ in einem Bereich von 0,7 - 1,4 aufweisen, wobei der Span $\Delta D$ gemäß der Formel $\Delta D = (D_{90} - D_{10}) / D_{50}$ definiert ist,
(ii) Aufbringen wenigstens der Schichten A bis C auf die plättchenförmigen transparenten Substrate sowie optional wenigstens einer Schicht D,
oder
(iii) Aufbringen wenigstens der Schichten A bis C auf die plättchenförmigen transparenten Substrate sowie optional wenigstens einer Schicht D,
(iv) Größenklassieren der zu beschichtenden plättchenförmigen transparenten Substrate, so dass die zu beschichtenden plättchenförmigen transparenten Substrate eine volumengemittelte Größenverteilungsfunktion mit den Kennzahlen $D_{10}$, $D_{50}$, $D_{90}$ und einen Span $\Delta D$ in einem Bereich von 0,7 - 1,4 aufweisen, wobei der Span $\Delta D$ gemäß der Formel $\Delta D = (D_{90} - D_{10}) / D_{50}$ definiert ist.

Gemäß einer bevorzugten Variante der Erfindung werden die zu beschichtenden plättchenförmigen transparenten Substrate zunächst gemäß Schritt (i) größenklassiert und sodann im Schritt (ii) wenigstens die Schichten A bis C und optional wenigstens eine Schicht D auf die plättchenförmigen transparenten Substrate aufgebracht.

Des Weiteren ist Gegenstand der Erfindung die Verwendung der erfindungsgemäßen Mehrschichtperlglanzpigmente in kosmetischen Formulierungen, Kunststoffen, Folien, Textilien, keramischen Materialien, Gläsern und Beschichtungszusammensetzungen wie Farben, Druckfarben, Lacken und Pulverlacken.

Die der Erfindung zugrundeliegende Aufgabe wird auch durch Bereitstellung eines Gegenstandes gelöst, wobei der Gegenstand die Mehrschichtperlglanzpigmente nach einem der Ansprüche 1 bis 10 enthält oder aufweist.

Gegenstand der Erfindung sind mithin ebenfalls Zubereitungen, wie kosmetische Formulierungen, Kunststoffe, keramische Materialien, Gläser oder Beschichtungszusammensetzungen wie Farben, Druckfarben, Lacke und Pulverlacke, die die erfindungsgemäßen Mehrschichtperlglanzpigmente enthalten. Die Erfindung ist auch auf Gegenstände gerichtet, die mit den erfindungsgemäßen Mehrschichtperlglanzpigmenten versehen, beispielsweise lackiert oder bedruckt, sind. Somit sind lackierte Gegenstände, wie Karosserien, Fassadenelemente, etc. oder bedruckte Gegenstände, wie Papier, Pappe, Folien, Textilien etc., ebenfalls Bestandteil der vorliegenden Erfindung.

Unter Mehrschichtperlglanzpigmenten mit verbessertem Glanz werden im Sinne dieser Erfindung mehrschichtige Perlglanzeffektpigmente verstanden, die auf einem transparenten plättchenförmigem Substrat wenigstens drei Beschichtungen aufweisen, wobei diese wenigstens drei Beschichtungen

> a) eine absorbierende hochbrechende Schicht A mit einem Brechungsindex $n \geq 1,8$,
> b) eine niedrigbrechende Schicht B mit einem Brechungsindex $n < 1,8$,
> c) eine hochbrechende Schicht C mit einem Brechungsindex $n \geq 1,8$

sind.

Geeignete zu beschichtende plättchenförmige transparente Substrate sind nichtmetallische, natürliche oder synthetische plättchenförmige Substrate. Die Substrate sind vorzugsweise im Wesentlichen transparent, bevorzugt transparent, d.h. für sichtbares Licht sind sie zumindest teilweise durchlässig.

Erfindungsgemäß ist die Schicht A in der Schichtenanordnung innenliegend, d.h. dem plättchenförmigen transparenten Substrat zugewandt, die Schicht B zwischen der Schicht A und der Schicht C liegend, und die Schicht C, bezogen auf das plättchenförmige transparente Substrat, in der Schichtenanordnung außenliegend.

Zwischen dem plättchenförmigen transparenten Substrat und der Schicht A können eine oder mehrere weitere, vorzugsweise im Wesentlichen transparente Schichten, angeordnet sein. Gemäß einer bevorzugten Weiterbildung ist die Schicht A direkt auf dem transparenten plättchenförmigem Substrat aufgebracht.

Zwischen der Schicht A und der Schicht B sowie zwischen der Schicht B und der Schicht C können unabhängig voneinander eine oder mehrere weitere, vorzugsweise im Wesentlichen transparente Schichten, angeordnet sein. Gemäß einer bevorzugten Weiterbildung ist die Schicht B direkt auf der Schicht A aufgebracht. Gemäß einer weiteren bevorzugten Weiterbildung ist die Schicht C direkt auf der Schicht B aufgebracht.

Äußerst bevorzugt ist die Schicht A unmittelbar auf dem plättchenförmigen transparenten Substrat, die Schicht B unmittelbar auf der Schicht A, die Schicht C unmittelbar auf der Schicht B sowie optional die Schicht D unmittelbar auf der Schicht C aufgebracht.

Die Erfinder haben überraschend festgestellt, dass die erfindungsgemäßen Mehrschichtperlglanzpigmente, d.h. Perlglanzpigmente mit der angegebenen Schichtenanordnung und einem Span $\Delta D$ gemäß Formel I im Bereich von 0,7 bis 1,4 einen außerordentlich starken Glanz aufweisen. Gemäß einer weiteren Variante der Erfindung weisen die erfindungsgemäßen Mehrschichtperlglanzpigmente bei einem Beobachtungswinkel nahe dem Glanzwinkel, vorzugsweise 15°, zugleich ein hohes Chroma auf.

Die Bereitstellung von Mehrschichtperlglanzpigmenten mit starkem Glanz und vorzugsweise zugleich auch hohem Chroma ist im Stand der Technik bislang nicht möglich gewesen.

Dass der Span $\Delta D$, wie in Anspruch 1 definiert, einen Einfluss auf den Glanz haben könnte, ist im Hinblick auf den veröffentlichten Stand der Technik unerwartet. Somit ermöglicht die vorliegende Erfindung die Bereitstellung von Mehrschichtperlglanzpigmenten, die äußerst ansprechende optische Eigenschaften aufweisen.

Im Stand der Technik wurde bislang der Span $\Delta D$ nicht als ein wesentliches Merkmal erkannt. Mithin weisen herkömmliche Mehrschichtperlglanzpigmente einen breiten Span auf.

Zur Charakterisierung der Größenverteilung der Mehrschichtperlglanzpigmente wird erfindungsgemäß der Span $\Delta D$, definiert als $\Delta D = (D_{90} - D_{10})/D_{50}$ (Formel I), verwendet. Je kleiner der Span ist, desto enger ist die Größenverteilung.

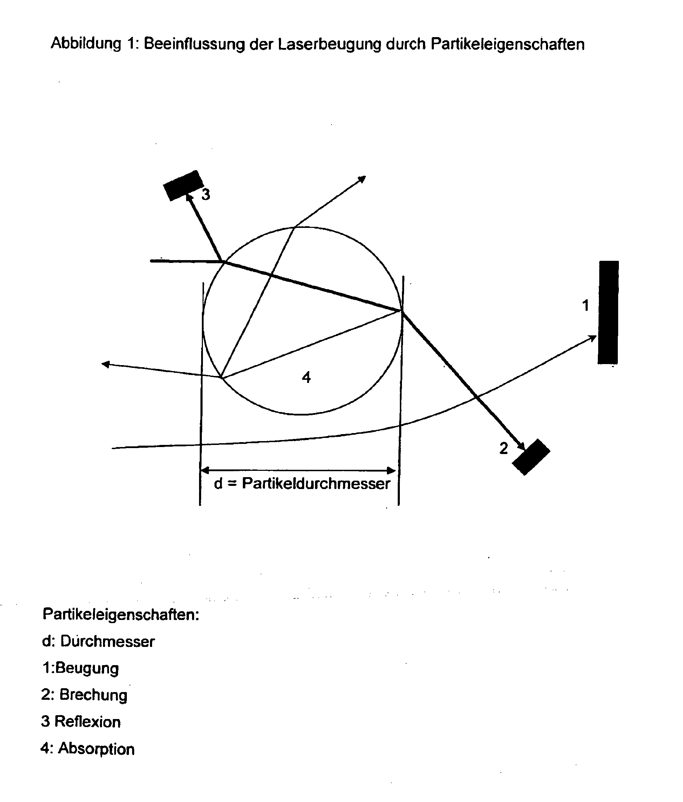

Der $D_{10}$-, $D_{50}$- bzw. $D_{90}$-Wert der Summenhäufigkeitsverteilung der volumengemittelten Größenverteilungsfunktion, wie sie durch Laserbeugungsmethoden erhalten wird, gibt an, dass 10%, 50% bzw. 90% der Mehrschichtperlglanzpigmente einen Durchmesser aufweisen, der gleich oder kleiner als der jeweils angegebene Wert ist. Hierbei wird die Größenverteilungskurve mit einem Gerät der Fa. Malvern (Gerät: MALVERN Mastersizer 2000) gemäß Herstellerangaben bestimmt. Die Auswertung der Streulichtsignale erfolgte dabei nach der Mie-Theorie, welche auch Brechungs- und Absorptionsverhalten der Partikel beinhaltet (Abbildung 1).

Die erfindungsgemäßen Mehrschichtperlglanzpigmente besitzen einen Span $\Delta D$ in einem Bereich von 0,7 bis 1,4, vorzugsweise von in einem Bereich von 0,7 bis 1,3, weiterhin bevorzugt in einem Bereich von 0,8 bis 1,2, besonders bevorzugt in einem Bereich von 0,8 bis 1,1. Bei weiterhin bevorzugten Ausführungsformen liegt der Span $\Delta D$ in einem

Bereich von 0,85 bis 1,05.

Weisen die Mehrschichtperlglanzpigmente einen Span $\Delta D$ oberhalb von 1,4 auf, so werden keine hochglänzenden Mehrschichtperlglanzpigmente erhalten. Mehrschichtperlglanzpigmente unterhalb eines Spans $\Delta D$ von 0,7 können im Rahmen der üblichen Methoden nur sehr aufwändig und damit nicht mehr wirtschaftlich hergestellt werden.

Der Span $\Delta D$ des zu beschichtenden, plättchenförmigen transparenten Substrats entspricht im Wesentlichen dem des erfindungsgemäßen Mehrschichtperlglanzpigments und liegt bei $\leq 1,4$, vorzugsweise $\leq 1,3$, weiter bevorzugt $\leq 1,2$, besonders bevorzugt $\leq 1,1$ und ganz besonders bevorzugt bei $\leq 1,05$.

Die erfindungsgemäßen Mehrschichtperlglanzpigmente können eine beliebige mittlere Teilchengröße ($D_{50}$) aufweisen. Die $D_{50}$-Werte der erfindungsgemäßen Mehrschichtperlglanzpigmente liegen vorzugsweise in einem Bereich von 3 bis 350 $\mu$m. Bevorzugt weisen die erfindungsgemäßen Mehrschichtperlglanzpigmente einen $D_{90}$-Wert aus einem Bereich von 3 bis 15 $\mu$m oder aus einem Bereich von 10 bis 35 $\mu$m oder aus einem Bereich von 25 bis 45 $\mu$m oder aus einem Bereich von 30 bis 65 $\mu$m oder aus einem Bereich von 40 bis 140 $\mu$m oder aus einem Bereich von 135 bis 250 $\mu$m auf.

Die $D_{10}$-Werte der erfindungsgemäßen Mehrschichtperlglanzpigmente umfassen vorzugsweise einen Bereich von 1 bis 120 $\mu$m. Bevorzugt weisen die erfindungsgemäßen Mehrschichtperlglanzpigmente die in Tabelle 1 angegebenen Kombinationen von $D_{10}$-, $D_{50}$- und $D_{90}$-Werten auf. Hierbei werden die $D_{10}$, $D_{50}$ und $D_{90}$-Werte der Tabelle 1 nur in der Art kombiniert, dass sich ein Span $\Delta D$ aus einem Bereich von 0,7 bis 1,4, vorzugsweise aus einem Bereich von 0,7 bis 1,3, weiterhin bevorzugt aus einem Bereich von 0,8 bis 1,2, besonders bevorzugt aus einem Bereich von 0,8 bis 1,1 und ganz besonders bevorzugt aus einem Bereich von 0,85 bis 1,05 ergibt. Kombinationen von $D_{10}$-, $D_{50}$- und $D_{90}$-Werten, die zu einem Span $\Delta D$ führen, der nicht im Bereich $\Delta D$ von 0,7 bis 1,4 liegt, sind keine erfindungsgemäßen Ausführungsformen.

Tabelle 1: Bevorzugte Kombinationen von Bereichen der $D_{10}$-, $D_{50}$- und $D_{90}$-Werte

| $D_{10}$ ($\mu$m) | $D_{50}$ ($\mu$m) | $D_{90}$ ($\mu$m) |
| --- | --- | --- |
| 1 - 5 | 3 - 15 | 8 - 25 |
| 5 - 25 | 10 - 35 | 20 - 45 |
| 10 - 30 | 25 - 45 | 40 - 70 |
| 20 - 45 | 30 - 65 | 70 - 110 |
| 25 - 65 | 40 - 140 | 120 - 80 |
| 75 - 110 | 135 - 250 | 400 - 490 |

Dabei hat sich überraschenderweise gezeigt, dass die Größe der Mehrschichtperlglanzpigmente, charakterisiert durch den $D_{50}$- Wert, nicht entscheidend ist, sondern dass der Span $\Delta D = (D_{90} - D_{10})/ D_{50}$ in einem engen Bereich von 0,7 bis 1,4 liegt. Dabei können beispielsweise die $D_{50}$-Werte der Mehrschichtperlglanzpigmente bei 15, 20, 25, 30 $\mu$m oder auch bei 50, 80, 100, 150, 200, 250, 300 oder 350 $\mu$m liegen.

Gemäß einer bevorzugten Ausführungsform der Erfindung weisen die Mehrschichtperlglanzpigmente nur eine (Zahl: 1) Interferenzfarbe auf. Bei dieser Variante der Erfindung kommt es mithin zu im Wesentlichen keinem winkelabhängigen Wechsel zwischen zwei oder mehr Interferenzfarben. Bei Änderung des Betrachtungswinkels ändert sich die Helligkeit der Interferenzfarbe, beispielsweise von hell nach dunkel oder umgekehrt, es kommt dabei jedoch nicht zu einem Wechsel der Interferenzfarbe. Die erfindungsgemäßen Mehrschichtperlglanzpigmente gemäß dieser Variante weisen mithin keinen winkelabhängigen Interferenzfarbenwechsel auf. Wenn in einer Lack- oder Farbschicht neben den erfindungsgemäßen Mehrschichtperlglanzpigmenten gemäß dieser Variante noch weitere Farbpigmente enthalten sind, kann sich der Farbeindruck winkelabhängig ändern, wobei dies jedoch kein Wechsel zwischen Interferenzfarben ist, sondern ein Wechsel zwischen einer Interferenzfarbe und der Absorptionsfarbe der weiter enthaltenen Farbpigmente.

Gemäß einer weiteren bevorzugten Ausführungsform der Erfindung weisen die Mehrschichtperlglanzpigmente wenigstens zwei Interferenzfarben auf. Bei dieser Variante der Erfindung kommt es mithin zu einem winkelabhängigen Wechsel zwischen zwei oder mehr Interferenzfarben. Bei Änderung des Betrachtungswinkels ändert sich mithin die Interferenzfarbe, beispielsweise von rot nach grün. Die erfindungsgemäßen Mehrschichtperlglanzpigmente gemäß dieser Variante weisen mithin einen winkelabhängigen Interferenzfarbenwechsel auf und können auch als goniochromatische Mehrschichtperlglanzpigmente bezeichnet werden.

Gemäß einer weiteren bevorzugten Ausführungsform der Erfindung weisen die erfindungsgemäßen Mehrschichtperlglanzpigmente keine silberne Interferenzfarbe auf. Die erfindungsgemäßen Mehrschichtperlglanzpigmente zeichnen sich dadurch aus, dass diese vorzugsweise bei einem glanznahen Betrachtungswinkel farbig, jedoch nicht silberfarben sind. Die erfindungsgemäßen Mehrschichtperlglanzpigmente unterscheiden sich mithin signifikant sowohl von silberfarbenen Interferenzpigmenten als auch von Metalleffektpigmenten, insbesondere von z.B. silberfarbenen Aluminiumeffektpigmenten.

Vorzugsweise weisen die erfindungsgemäßen Mehrschichtperlglanzpigmente wenigstens eine Interferenzfarbe auf, die aus der Gruppe ausgewählt wird, die aus gelb, violett, blau, rot, grün und Abstufungen davon besteht, jedoch keine silberne Interferenzfarbe umfasst. Die jeweilige Interferenzfarbe kann dabei von dunkel bis hell reichen.

Unter Mehrschichtperlglanzpigmenten mit nichtsilberner Interferenzfarbe werden im Rahmen dieser Erfindung Mehrschichtperlglanzpigmente verstanden, deren Chromawerte $C^*_{15} > 20$ sind.

Die Chromawerte werden dabei anhand von folgenden Applikationen ermittelt: Ein Nitrocelluloselack (Dr. Renger Erco Bronzemischlack 2615e; Fa. Morton), der 6 Gew.-% an Mehrschichtperlglanzpigmenten enthält, wobei sich die Gew.-% Angabe auf das Gesamtgewicht des Lackes bezieht, wird je nach $D_{50}$-Wert in einer Nassfilmdicke gemäß Tabelle 2 auf BYK-Gardner Schwarz-Weiß-Rakelkarten (Byko-Chart 2853) aufgetragen und nachfolgend bei Raumtemperatur getrocknet. An diesen Rakelkarten werden dann mit einem BYK-MAC farbmetrische Auswertungen vorgenommen, wobei auf dem schwarzen Untergrund der Rakelkarte vermessen wird. Der Einfallswinkel beträgt 45° und herangezogen wird der Chromawert bei einem Beobachtungswinkel von 15°.

Tabelle 2: Nassfilmdicke in Abhängigkeit vom $D_{90}$-Wert der Mehrschichtperlglanzpigmente

| $D_{50}$-Wert | Spiralrakel |
|---|---|
| <40 $\mu m$ | 36 $\mu m$ |
| 40 $\mu m$ - 85 $\mu m$ | 76 $\mu m$ |
| > 85 $\mu m$ | 100 $\mu m$ |

Die erfindungsgemäßen Mehrschichtperlglanzpigmente zeichnen sich durch einen starken Glanz sowie vorzugsweise zugleich durch ein hohes Chroma $C^*_{15} > 20$ aus.

Gemäß einer bevorzugten Variante der Erfindung beträgt das Chroma $C^*_{15}$ der erfindungsgemäßen Mehrschichtperlglanzpigmente wenigstens 22, vorzugsweise wenigstens 24, noch weiter bevorzugt wenigstens 25. Als sehr geeignet hat sich ein Chroma in einem Bereich von 24 bis 50 erwiesen.

Geeignete zu beschichtende plättchenförmige transparente Substrate sind nichtmetallische, natürliche oder synthetische plättchenförmige Substrate. Die Substrate sind vorzugsweise im Wesentlichen transparent, bevorzugt transparent, d.h. für sichtbares Licht sind sie zumindest teilweise durchlässig.

Gemäß einer bevorzugten Ausführungsform der Erfindung können die plättchenförmigen transparenten Substrate aus der Gruppe, bestehend aus natürlichem Glimmer, synthetischem Glimmer, Glasflakes, $SiO_2$-Plättchen, $Al_2O_3$-Plättchen, Polymerplättchen, plättchenförmigem Bismuthoxychlorid, plättchenförmigen Substraten umfassend eine anorganisch-organische Mischschicht und deren Gemische, ausgewählt werden. Bevorzugt werden die plättchenförmigen transparenten Substrate aus der Gruppe, bestehend aus natürlichem Glimmer, synthetischem Glimmer, Glasflakes, $SiO_2$-Plättchen, $Al_2O_3$-Plättchen und deren Gemische, ausgewählt. Besonders bevorzugt werden die plättchenförmigen transparenten Substrate aus der Gruppe, bestehend aus natürlichem Glimmer, synthetischem Glimmer, Glasflakes und deren Gemische, ausgewählt. Ganz besonders bevorzugt sind Glasflakes und synthetischer Glimmer und deren Gemische.

Natürlicher Glimmer besitzt im Unterschied zu synthetischen plättchenförmigen transparenten Substraten den Nachteil, dass Verunreinigungen durch eingelagerte Fremdionen den Farbton verändern können, und dass die Oberfläche nicht ideal glatt ist, sondern Unregelmäßigkeiten, wie z.B. Stufen aufweisen kann.

Jedoch auch bei Verwendung von natürlichem Substrat hat sich überraschenderweise gezeigt, dass der Glanz einer Mehrzahl von Mehrschichtperlganzpigmenten gesteigert werden kann, wenn der Span $\Delta D$ in einem Bereich von 0,7 bis 1,4 liegt, verglichen mit einer Mehrzahl an herkömmlichen Mehrschichtperlglanzpigmenten mit breitem Span.

Synthetische Substrate wie beispielsweise Glasflakes oder synthetischer Glimmer dagegen weisen glatte Oberflächen, eine einheitliche Dicke innerhalb eines einzelnen Substratteilchens sowie scharfe Kanten auf. Somit bietet die Oberfläche nur wenig Streuzentren für einfallendes bzw. reflektiertes Licht und ermöglicht so nach Beschichtung höher glänzende Mehrschichtperlglanzpigmente als mit natürlichem Glimmer als Substrat. Dadurch resultieren sehr einheitliche Farben, wenn sie erfindungsgemäß mit hoch- und niedrigbrechenden Schichten beschichtet werden. Alle diese Effekte tragen vorteilhaft dazu bei, hochchromatische mehrschichtige Perlglanzpigmente mit ausgeprägtem Glanz zu erhalten. Als Glasflakes werden bevorzugt jene verwendet, die nach den in EP 0 289 240 A1, WO 2004/056716 A1 und der WO 2005/063637 A1 beschriebenen Verfahren hergestellt werden. Die als Substrat verwendbaren Glasflakes können beispielsweise eine Zusammensetzung entsprechend der Lehre der EP 1 980 594 B1 aufweisen.

Die mittlere geometrische Dicke der zu beschichtenden plättchenförmigen transparenten Substrate liegt in einem Bereich von 50 nm bis 5000 nm, bevorzugt in einem Bereich von 60 nm bis 3000 nm und besonders bevorzugt in einem Bereich von 70 nm bis 2000 nm. In einer Ausführungsform liegt die mittlere geometrische Dicke für Glasflakes als zu beschichtendes Substrat in einem Bereich von 750 nm bis 1500 nm. Derartige Glasflakes sind kommerziell auf breiter Basis verfügbar. Weitere Vorteile bieten dünnere Glasflakes. Dünnere Substrate führen zu einer geringeren Gesamtschichtdicke der erfindungsgemäßen Mehrschichtperlglanzpigmente. So sind ebenfalls bevorzugt Glasflakes deren mittleren geometrischen Dicke in einem Bereich von 100 nm bis 700 nm, weiter bevorzugt in einem Bereich von 150 nm bis 600 nm, besonders bevorzugt in einem Bereich von 170 nm bis 500 nm und ganz besonders bevorzugt in einem Bereich von 200 nm bis 400 nm liegt.

In einer weiteren Ausführungsform liegt die mittlere geometrische Dicke für natürlichen oder synthetischen Glimmer als zu beschichtendes Substrat bevorzugt in einem Bereich von 100 nm bis 700 nm, weiter bevorzugt in einem Bereich von 150 nm bis 600 nm, besonders bevorzugt in einem Bereich von 170 nm bis 500 nm und ganz besonders bevorzugt in einem Bereich von 200 nm bis 400 nm.

Beschichtet man plättchenförmige transparente Substrate unterhalb einer mittleren geometrischen Dicke von 50 nm mit hochbrechenden Metalloxiden, so werden extrem bruchempfindliche Mehrschichtperlglanzpigmente erhalten, die schon beim Einarbeiten in das Anwendungsmedium zerbrechen können, was wiederum eine signifikante Herabsetzung des Glanzes bedingt.

Oberhalb einer mittleren geometrischen Substratdicke von 5000 nm können die Mehrschichtperlglanzpigmente insgesamt zu dick werden. Damit geht eine schlechtere spezifische Deckfähigkeit, d.h. abgedeckte Fläche pro Gewichtseinheit an erfindungsgemäßem Mehrschichtperlglanzpigment, einher sowie eine geringere planparallele Orientierung im Anwendungsmedium. Aus einer schlechteren Orientierung wiederum resultiert ein verminderter Glanz.

Die mittlere geometrische Dicke des plättchenförmigen transparenten Substrates wird anhand eines gehärteten Lackfilmes, in dem die Mehrschichtperlglanzpigmente im Wesentlichen planparallel zum Untergrund ausgerichtet sind, bestimmt. Hierzu wird ein Querschliff des gehärteten Lackfilmes unter einem Rasterelektronenmikroskop (REM) untersucht, wobei die geometrische Dicke des plättchenförmigen transparenten Substrates von 100 Mehrschichtperlglanzpigmenten bestimmt und statistisch gemittelt wird.

Bei den erfindungsgemäßen Mehrschichtpigmenten werden die optischen Wirkungen durch den Schichtenaufbau mit den Schichten A bis C hervorgerufen, an dem einfallendes Licht durch physikalische Effekte wie Reflexion, Interferenz, Absorption, Lichtbrechung, etc. die wahrnehmbaren Farbeffekte erzeugt.

Als optische wirksame Schichten oder Beschichtungen werden vorzugsweise Schichten aufgebracht, die Metalloxide, Metalloxidhydrate, Metallhydroxide, Metallsuboxide, Metalle, Metallfluoride, Metalloxyhalide, Metallchalcogenide, Metallnitride, Metalloxynitride, Metallsulfide, Metallcarbide oder Mischungen davon umfassen. Gemäß einer bevorzugten Variante bestehen die optisch wirksamen Schichten oder Beschichtungen aus den vorstehend genannten Materialien.

Die Begriffe Schichten oder Beschichtungen werden im Sinne dieser Erfindung austauschbar verwendet, sofern nicht anders angegeben.

Der Brechungsindex der hochbrechenden Schichten A und C liegt jeweils bei n ≥ 1,8, bevorzugt bei n ≥ 1,9 und besonders bevorzugt bei n ≥ 2,0. Der Brechungsindex der niedrigbrechenden Schicht B liegt bei n < 1,8, bevorzugt bei n < 1,7 und besonders bevorzugt bei n < 1,6.

Erfindungsgemäß ist die Schicht A eine absorbierende hochbrechende Schicht. Die Schicht A kann dabei selektiv, d.h. in einem engen Wellenlängenbereich, absorbierend sein. Die Schicht A kann dabei auch nicht selektiv absorbierend, d.h. über den gesamten Wellenlängenbereich des sichtbaren Lichts absorbierend sein.

Die Schicht C kann bei den erfindungsgemäßen Mehrschichtperlglanzpigmenten absorbierend oder nicht absorbierend sein. Wenn die Schicht C absorbierend ist, kann die Schicht C selektiv absorbierend oder nicht selektiv absorbierend sein, wie vorstehend im Hinblick auf die Schicht A ausgeführt. Die Schicht C kann dabei eine identische chemische Zusammensetzung wie die Schicht A aufweisen. Die Schicht C kann aber auch eine chemische Zusammensetzung aufweisen, die von der Schicht A verschieden ist.

Eine nicht absorbierende Schicht C kann auch als transparente Schicht bezeichnet werden.

Als hochbrechende selektiv absorbierende Materialien eignen sich beispielsweise

■ farbige Metalloxide oder Metalloxidhydrate wie Eisen(III)oxid ($\alpha$- und/ oder $\gamma$-$Fe_2O_3$, rot), $FeO(OH)$ (gelb), Chrom(III)oxid (grün), Titan(III)oxid ($Ti_2O_3$, blau), Vanadiumpentoxid (orange),

■ farbige Nitride wie Titanoxynitrid und Titannitrid ($TiO_xN_y$, TiN, blau), wobei die niederen Titanoxide und -nitride in der Regel im Gemisch mit Titandioxid vorliegen,

■ Metallsulfide wie Cersulfid (rot),

■ Eisentitanate wie Pseudobrookit (braunrot) und/ oder Pseudorutil (braunrot),

■ Zinn-Antimonoxid $Sn(Sb)O_2$,

■ nicht absorbierende farblose hochbrechende Materialien, z. B. Metalloxide wie Titandioxid und Zirkoniumdioxid, die mit selektiv absorbierenden Farbmiteln eingefärbt sind. Diese Einfärbung kann durch Einbau von Farbmitteln in die Metalloxidschicht, durch deren Dotierung mit selektiv absorbierenden Metallkationen oder farbigen Metalloxiden wie Eisen(III)oxid oder durch Überziehen der Metalloxidschicht mit einem ein Farbmittel enthaltenden Film erfolgen.

Beispiele für hochbrechende nichtselektiv absorbierende Materialien sind u.a.

■ Metalle wie Molybdän, Eisen, Wolfram, Chrom, Cobalt, Nickel, Silber, Palladium, Platin, deren Gemische oder Legierungen,
■ Metalloxide wie Magnetit $Fe_3O_4$, Cobaltoxid (CoO und/ oder $Co_3O_4$), Vanadiumoxid ($VO_2$ und/ oder $V_2O_3$) sowie auch Mischungen dieser Oxide mit Metallen, insbesondere Magnetit und (metallischem) Eisen,
■ Eisentitanante wie Ilmenit,
■ Metallsulfide wie Moybdänsulfid, Eisensulfid, Wolframsulfid, Chromsulfid, Colbaltsulfid, Nickelsulfid, Silbersulfid, Zinnsulfid, Gemische dieser Sulfide, Gemische dieser Sulfide mit dem jeweiligen Metall, wie $MoS_2$ und Mo, und Gemische mit Oxiden des jeweiligen Metalls, wie $MoS_2$ und Molybdänoxide,
■ nicht absorbierende farblose hochbrechende Schichten wie Titandioxid oder Zirkoniumdioxid, in die nichtselektiv absorbierendes Material (z.B. Kohlenstoff) eingebaut ist oder die damit beschichtet sind.

Zu den hochbrechenden nicht absorbierenden Materialien zählen beispielsweise

■ Metalloxide wie Titandioxid, Zirkoniumdioxid, Zinkoxid, Zinndioxid, Antimonoxid und deren Gemische,
■ Metallhydroxide
■ Metalloxidhydrate
■ Metallsulfide wie Zinksulfid,
■ Metalloxyhalide wie Bismutoxychlorid.

Die Schichten A und/ oder C können jeweils auch Mischungen verschiedener selektiv und/ oder nicht selektiv absorbierender Komponenten, vorzugsweise Metalloxide, sein. Beispielsweise können die verschiedenen Komponenten, vorzugsweise Metalloxide, als homogene Mischung vorliegen. Es ist aber auch möglich, dass die eine Komponente in der anderen Komponente in Form einer Dotierung vorliegt.

Beispielsweise kann in der Schicht A und/ oder C eine nicht absorbierende Komponente, beispielsweise Titanoxid, vorzugsweise $TiO_2$, als Dotierung in einer selektiv absorbierenden Komponente, vorzugsweise $Fe_2O_3$, und/ oder einer nicht selektiv absorbierenden Komponente, beispielsweise $Fe_3O_4$, vorliegen. Alternativ kann auch eine selektiv absorbierende Komponente, beispielsweise $Fe_2O_3$, und/ oder eine nicht selektiv absorbierende Komponente, beispielsweise $Fe_3O_4$, als Dotierung in einer nicht absorbierenden Komponente, beispielsweise Titanoxid, vorzugsweise $TiO_2$, vorliegen.

Auch ist es selbstverständlich möglich, dass Gemische von mehr als zwei Komponenten, wie vorstehend veranschaulicht, in der Schicht A und/ oder C vorliegen.

In einer bevorzugten Ausführungsform werden als hochbrechende Schicht A bzw. C Metalloxide, Metallhydroxide und/ oder Metalloxidhydrate verwendet. Besonders bevorzugt werden Metalloxide eingesetzt. Ganz besonders bevorzugt umfasst die Schicht A Eisenoxid und die Schicht C Titandioxid und/ oder Eisenoxid sowie Mischungen davon. In einer Ausführungsform besteht die Schicht A aus Eisenoxid und die Schicht C aus Titandioxid und/ oder Eisenoxid sowie Mischungen davon.

Weisen die erfindungsgemäßen Mehrschichtperlglanzpigmente eine Beschichtung mit Titandioxid auf, kann das Titandioxid in der Rutil- oder Anataskristallmodihkation vorliegen. Vorzugsweise liegt die Titandioxidschicht in der Rutilform vor. Die Rutilform kann erhalten werden, indem beispielsweise vor Aufbringung der Titandioxidschicht eine Schicht aus Zinndioxid auf das zu beschichtende plättchenförmige transparente Substrat aufgebracht wird. Auf dieser Schicht aus Zinndioxid kristallisiert Titandioxid in der Rutilmodifikation. Das Zinnoxid kann dabei als separate Schicht vorliegen, wobei die Schichtdicke wenige Nanometer, beispielsweise weniger als 10 nm, weiter bevorzugt weniger als 5 nm, noch weiter bevorzugt weniger als 3 nm, betragen kann.

Als niedrigbrechende Schicht B eignen sich nicht absorbierende Materialien. Hierzu zählen beispielsweise

■ Metalloxide wie Siliziumdioxid, Aluminiumoxid, Boroxid,
■ Metalloxidhydrate wie Siliziumoxidhydrat, Aluminiumoxidhydrat,
■ Metallfluoride wie Magnesiumfluorid,
■ $MgSiO_3$.

Gegebenenfalls kann die niedrigbrechende Metalloxidschicht Alkali- und/ oder Erdalkalioxide als Bestandteile enthalten.

Vorzugsweise umfasst die niedrigbrechende Schicht B Siliziumdioxid. In einer Ausführungsform besteht die niedrigbrechende Schicht B aus Siliziumdioxid.

Die interferenzfähige Beschichtung kann entweder das Substrat vollständig umhüllen oder nur partiell auf dem Substrat vorliegen. Die erfindungsgemäßen Mehrschichtperlglanzpigmente zeichnen sich durch den gleichmäßigen homogenen Aufbau der Beschichtung aus, welche das plättchenförmige Substrat vollständig umhüllt und nicht nur dessen Ober- und Unterseite bedeckt.

Die einzelnen Schichten der erfindungsgemäßen Mehrschichtperlglanzpigmente können jeweils als λ/4-Schichten ausgebildet sein. Es hat sich jedoch überraschend herausgestellt, dass es nicht erforderlich ist, dass die Schichten als λ/4-Schichten ausgebildet sein müssen, um glanzstarke und vorzugsweise auch hochchromatische Mehrschichtperlglanzpigmente zu erhalten. Der entscheidende Parameter ist der Span der Größenverteilung, um glanzstarke und vorzugsweise auch Mehrschichtperlglanzpigmente mit hohem Chroma zu erhalten.

Die optische Dicke der nichtmetallischen Schichten mit hoher und niedriger Brechzahl bestimmt die optischen Eigenschaften der Mehrschichtperlglanzpigmente. Die Anzahl und Dicke der Schichten kann in Abhängigkeit vom gewünschten Effekt und vom verwendeten Substrat eingestellt werden.

Wenn n die Brechzahl einer Schicht und d ihre Dicke ist, ergibt sich die Interferenzfarbe, in der eine dünne Schicht erscheint, aus dem Produkt von n und d, d.h. der optischen Dicke. Die bei Normallichteinfall im reflektierenden Licht entstehenden Farben eines solchen Filmes ergeben sich aus einer Verstärkung des Lichtes der Wellenlänge

$$\lambda = \frac{4}{2N-1} \cdot nd$$

und durch Schwächung von Licht der Wellenlänge

$$\lambda = \frac{2}{N} \cdot nd \ ,$$

wobei *N* eine positive ganze Zahl ist. Die bei zunehmender Filmdicke erfolgende Variation der Farbe ergibt sich aus der Verstärkung bzw. Schwächung bestimmter Wellenlängen des Lichtes durch Interferenz.

Bei mehrschichtigen Pigmenten wird die Interferenzfarbe durch die Verstärkung bestimmter Wellenlängen bestimmt und wenn mehrere Schichten in einem vielschichtigen Pigment die gleiche optische Dicke besitzen, wird die Farbe des reflektierenden Lichtes mit zunehmender Zahl der Schichten intensiver. Darüber hinaus kann durch geeignete Wahl der Schichtdicke der niedrigbrechenden Schicht B eine besonders starke Variation der Farbe in Abhängigkeit vom Betrachtungswinkel erreicht werden. Es kann sich somit ein ausgeprägter Farbflop ausbilden.

Die erfindungsgemäßen Mehrschichtperlglanzpigmente können optische Schichtdicken der hochbrechenden Schichten A und C aufweisen, die jeweils im Bereich von 30 nm bis 900 nm, bevorzugt im Bereich von 40 nm bis 880 nm und besonders bevorzugt im Bereich von 50 nm bis 850 nm liegen. Die optische Schichtdicke der niedrigbrechenden Schicht B kann in einem Bereich von 30 nm bis 500 nm liegen.

Im Fall von Metallen liegen die geometrischen Schichtdicken der Schicht A bzw. C bei 10 nm bis 50 nm, bevorzugt bei 15 nm bis 30 nm. Die Schichtdicken müssen so eingestellt werden, dass die Schichten semitransparente Eigenschaften aufweisen. Abhängig vom verwendeten Metall kann das zu unterschiedlichen Schichtdicken führen.

Bei den in dieser Anmeldung angegeben Schichtdicken handelt es sich - sofern nicht anders angegeben - um die optische Schichtdicken. Unter einer optischen Schichtdicke wird erfindungsgemäß das Produkt aus geometrischer Schichtdicke und dem Brechungsindex des die Schicht aufbauenden Materials verstanden. Als Wert für den Brechungsindex des jeweiligen Materials wird der aus der Literatur jeweils bekannte Wert verwendet. Die geometrische Schichtdicke wird dabei erfindungsgemäß anhand von Querschliffaufnahmen von Lacken, in denen planparallel zum Untergrund orientierte Mehrschichtperlglanzpigmente vorliegen, mittels REM bestimmt.

Bevorzugte Weiterbildungen der Erfindung umfassen folgende Schichtenfolgen, die auf dem plättchenförmigen transparenten Substrat, beginnend mit der Schicht A, dann Schicht B und schließlich Schicht C, aufgebracht sind:

1. Schicht A: selektiv absorbierend und hochbrechend
Schicht B: niedrigbrechend
Schicht C: selektiv absorbierend und hochbrechend

2. Schicht A: nicht selektiv absorbierend und hochbrechend
Schicht B: niedrigbrechend
Schicht C: selektiv absorbierend und hochbrechend

3. Schicht A: selektiv absorbierend und hochbrechend
Schicht B: niedrigbrechend
Schicht C: nicht selektiv absorbierend und hochbrechend

4. Schicht A: nicht selektiv absorbierend und hochbrechend
Schicht B: niedrigbrechend
Schicht C: nicht selektiv absorbierend und hochbrechend

5. Schicht A: selektiv absorbierend und hochbrechend
Schicht B: niedrigbrechend
Schicht C: nicht absorbierend (transparent) und hochbrechend

6. Schicht A: nicht selektiv absorbierend und hochbrechend
Schicht B: niedrigbrechend
Schicht C: nicht absorbierend (transparent) und hochbrechend

Die vorstehend genannten Schichtenfolgen können jeweils noch wenigstens eine äußere Schutzschicht D, die optional organochemisch modifiziert ist, aufweisen.

Gemäß einer bevorzugten Weiterbildung der vorliegenden Erfindung weist bei den vorstehend genannten Schichtenfolgen die Schicht B eine optische Schichtdicke von $\leq$ 150 nm, vorzugsweise von < 140 nm, noch weiter bevorzugt von < 130 nm, auf. Eine optische Schichtdicke der Schicht B im Bereich von 30 nm bis $\leq$ 150 nm, bevorzugt in Bereich von 40 nm bis 140 nm und besonders bevorzugt im Bereich von 50 nm bis 130 nm, hat sich als sehr geeignet erwiesen.

Wenn die optische Schichtdicke der Schicht B $\leq$ 150 nm ist, weisen die erfindungsgemäßen Mehrschichtperlglanzpigmente im Wesentlichen keine winkelabhängige Interferenzfarbe auf. Bei dieser Ausführungsform weisen die erfindungsgemäßen Mehrschichtperlglanzpigmente nur eine Interferenzfarbe auf, wobei sich deren Intensität von hell nach dunkel in Abhängigkeit vom Betrachtungswinkel ändert. Bei dieser Variante der erfindungsgemäßen Mehrschichtperlglanzpigmente werden mithin glanzstarke und hochchromatische Effektpigmente erhalten.

Gemäß einer weiteren bevorzugten Weiterbildung der vorliegenden Erfindung weist bei den vorstehend genannten Schichtenfolgen die Schicht B eine optische Schichtdicke von > 150 nm, vorzugsweise von > 180 nm, noch weiter bevorzugt von > 220 nm, auf. Eine optische Schichtdicke der Schicht B im Bereich von > 150 nm bis 500 nm, bevorzugt im Bereich von 180 nm bis 480 nm und besonders bevorzugt im Bereich von 220 nm bis 450 nm hat sich als sehr geeignet erwiesen.

Wenn die optische Schichtdicke der Schicht B > 150 nm ist, weisen die erfindungsgemäßen Mehrschichtperlglanzpigmente eine winkelabhängige Interferenzfarbe auf. Bei dieser Ausführungsform weisen die erfindungsgemäßen Mehrschichtperlglanzpigmente wenigstens zwei Interferenzfarben in Abhängigkeit vom Betrachtungswinkel auf. Bei dieser Ausführungsform der erfindungsgemäßen Mehrschichtperlglanzpigmente können diese auch als goniochromatische Perlglanzpigmente bezeichnet werden. Bei dieser Variante der erfindungsgemäßen Mehrschichtperlglanzpigmente werden mithin glanzstarke Perlglanzpigmente mit intensivem Farbflop erhalten. Beispielsweise können diese Mehrschichtperlglanzpigmente einen Interferenzfarbwechsel von rot nach grün oder von blau nach gelb aufweisen.

Der Übergang zwischen Mehrschichtperlglanzpigmenten mit keinem, schwachem und intensivem Farbflop in Abhängigkeit von der optischen Schichtdicke der niedrigbrechenden Schicht B verläuft fließend. Mit zunehmender optischer Schichtdicke der niedrigbrechenden Schicht B werden ab 150 nm zunächst Mehrschichtperlglanzpigmente erhalten, die nur einen schwachen Farbflop aufweisen, welcher letztendlich mit immer weiter steigender optischer Schichtdicke von Schicht B in einen intensiven Farbflop übergeht. Typischerweise erstreckt sich ein intensiver Farbflop über mehrere Quadranten im CIELab-Farbkoordinatensystem.

Gemäß einer bevorzugten Weiterbildung der Erfindung sind folgende Ausführungsformen besonders bevorzugt:

1. Schicht A: selektiv absorbierend und hochbrechend
Schicht B: niedrigbrechend, optische Schichtdicke $\leq$ 150 nm, vorzugsweise aus einem Bereich von 30 nm bis 140 nm
Schicht C: selektiv absorbierend und hochbrechend

2. Schicht A: nicht selektiv absorbierend und hochbrechend
Schicht B: niedrigbrechend, optische Schichtdicke $\leq$ 150 nm, vorzugsweise aus einem Bereich von 30 nm bis 140 nm
Schicht C: selektiv absorbierend und hochbrechend

3. Schicht A: selektiv absorbierend und hochbrechend
Schicht B: niedrigbrechend, optische Schichtdicke $\leq$ 150 nm, vorzugsweise aus einem Bereich von 30 nm bis 140 nm

Schicht C: nicht selektiv absorbierend und hochbrechend

4. Schicht A: nicht selektiv absorbierend und hochbrechend
Schicht B: niedrigbrechend, optische Schichtdicke $\leq$ 150 nm, vorzugsweise aus einem Bereich von 30 nm bis 140 nm
Schicht C: nicht selektiv absorbierend und hochbrechend

5. Schicht A: selektiv absorbierend und hochbrechend $\leq$
Schicht B: niedrigbrechend, optische Schichtdicke $\leq$ 150 nm, vorzugsweise aus einem Bereich von 30 nm bis 140 nm
Schicht C: nicht absorbierend (transparent) und hochbrechend

6. Schicht A: nicht selektiv absorbierend und hochbrechend
Schicht B: niedrigbrechend, optische Schichtdicke $\leq$ 150 nm, vorzugsweise aus einem Bereich von 30 nm bis 140 nm
Schicht C: nicht absorbierend (transparent) und hochbrechend

Die vorstehend genannten Schichtenfolgen können jeweils noch wenigstens eine äußere Schutzschicht D, die optional organochemisch modifiziert ist, aufweisen. Die optische Schichtdicke der Schicht B bei den vorstehend genannten sechs Ausführungsformen liegt gemäß einer weiteren bevorzugten Ausführungsform im Bereich von 40 nm bis 140 nm, noch weiter bevorzugt von 50 nm bis 130 nm. Vorzugsweise ist die Schicht B Siliziumoxid, bevorzugt $SiO_2$. Die Schicht A ist bevorzugt Eisenoxid, die Schicht C bevorzugt $TiO_2$ für transparenten Schichten und Eisenoxid für absorbierende Schichten.

Gemäß einer weiteren Variante der Erfindung werden Mehrschichtperlglanzpigmente bevorzugt, die eine winkelabhängige Änderung der Interferenzfarbe aufweisen. In Abhängigkeit vom Betrachtungswinkel weisen diese Mehrschichtperlglanzpigmente zwei oder mehr Interferenzfarben auf und können mithin als goniochromatische Mehrschichtperlglanzpigmente bezeichnet werden. Bevorzugte Ausführungsformen sind nachstehend angegeben:

1. Schicht A: selektiv absorbierend und hochbrechend
Schicht B: niedrigbrechend, optische Schichtdicke > 150 nm, bevorzugt aus einem Bereich von 165 nm bis 450 nm
Schicht C: selektiv absorbierend und hochbrechend

2. Schicht A: nicht selektiv absorbierend und hochbrechend
Schicht B: niedrigbrechend, optische Schichtdicke >150 nm, bevorzugt aus einem Bereich von 165 nm bis 450 nm
Schicht C: selektiv absorbierend und hochbrechend

3. Schicht A: selektiv absorbierend und hochbrechend
Schicht B: niedrigbrechend, optische Schichtdicke >150 nm, bevorzugt aus einem Bereich von 165 nm bis 450 nm
Schicht C: nicht selektiv absorbierend und hochbrechend

4. Schicht A: nicht selektiv absorbierend und hochbrechend
Schicht B: niedrigbrechend, optische Schichtdicke >150 nm, bevorzugt aus einem Bereich von 165 nm bis 450
Schicht C: nicht selektiv absorbierend und hochbrechend

5. Schicht A: selektiv absorbierend und hochbrechend
Schicht B: niedrigbrechend, optische Schichtdicke >150 nm, bevorzugt aus einem Bereich von 165 nm bis 450 nm
Schicht C: nicht absorbierend (transparent) und hochbrechend

6. Schicht A: nicht selektiv absorbierend und hochbrechend
Schicht B: niedrigbrechend, optische Schichtdicke >150 nm, bevorzugt 165 bis 450 nm
Schicht C: nicht absorbierend (transparent) und hochbrechend

Die optische Schichtdicke B der vorgenannten Mehrschichtperlglanzpigmente liegt vorzugsweise in einem Bereich von > 150 nm bis 500 nm, weiter bevorzugt von 180 nm bis 480 nm. Oberhalb einer optischen Schichtdicke von 500 nm wird das mehrschichtige Perlglanzpigment insgesamt zu dick. Dickere Perlglanzpigmente können sich schlechter im Anwendungsmedium planparallel orientieren und erleiden demzufolge auch einen Glanzverlust.

Vorzugsweise besteht die Schicht B aus Siliziumoxid, vorzugsweise $SiO_2$. Die Schicht A ist bevorzugt selektiv absorbierendes oder nicht selektiv absorbierendes Eisenoxid, die Schicht C bevorzugt $TiO_2$ für transparenten Schichten und Eisenoxid für absorbierende Schichten.

Erfindungsgemäß sind folgende spezielle Schichtenfolgen besonders bevorzugt:

i. Mehrschichtperiglanzpigmente mit nur einer (Zahl: 1) Interferenzfarbe, d.h. ohne winkelabhängigen Wechsel der Interferenzfarbe

Schicht A: selektiv absorbierendes Eisenoxid, vorzugsweise $Fe_2O_3$
Schicht B: Siliziumoxid, bevorzugt $SiO_2$, optische Schichtdicke $\leq$ 150 nm, bevorzugt aus einem Bereich von 40 nm bis 140 nm
Schicht C: Titanoxid, vorzugsweise Titandioxid
Schicht A: selektiv absorbierendes Eisenoxid, vorzugsweise $Fe_2O_3$
Schicht B: Siliziumoxid, bevorzugt $SiO_2$, optische Schichtdicke $\leq$ 150 nm, bevorzugt aus einem Bereich von 40 nm bis 140 nm
Schicht C: Titanoxid, vorzugsweise Titandioxid, und selektiv absorbierendes Eisenoxid, vorzugsweise $Fe_2O_3$,
[0097]   Schicht A: selektiv absorbierendes Eisenoxid, vorzugsweise $Fe_2O_3$
Schicht B: Siliziumoxid, bevorzugt $SiO_2$, optische Schichtdicke $\leq$ 150 nm, bevorzugt aus einem Bereich von 40 nm bis 140 nm
Schicht C: Titanoxid, vorzugsweise Titandioxid, und nicht selektiv absorbierendes Eisenoxid, vorzugsweise $Fe_3O_4$
Schicht A: selektiv absorbierendes Eisenoxid, vorzugsweise $Fe_2O_3$
Schicht B: Siliziumoxid, bevorzugt $SiO_2$, optische Schichtdicke $\leq$ 150 nm, bevorzugt aus einem Bereich von 40 nm bis 140 nm
Schicht C: nicht selektiv absorbierendes Eisenoxid, vorzugsweise $Fe_3O_4$ und selektiv absorbierendes Eisenoxid, vorzugsweise $Fe_2O_3$
Schicht A: selektiv absorbierendes Eisenoxid, vorzugsweise $Fe_2O_3$
Schicht B: Siliziumoxid, bevorzugt $SiO_2$, optische Schichtdicke $\leq$ 150 nm, bevorzugt aus einem Bereich von 40 nm bis 140 nm
Schicht C: selektiv absorbierendes Eisenoxid, vorzugsweise $Fe_2O_3$
Schicht A: nicht selektiv absorbierendes Eisenoxid, vorzugsweise $Fe_3O_4$
Schicht B: Siliziumoxid, bevorzugt $SiO_2$, optische Schichtdicke $\leq$ 150 nm, bevorzugt aus einem Bereich von 40 nm bis 140 nm
Schicht C: selektiv absorbierendes Eisenoxid, vorzugsweise $Fe_2O_3$
Schicht A: nicht selektiv absorbierendes Eisenoxid, vorzugsweise $Fe_3O_4$
Schicht B: Siliziumoxid, bevorzugt $SiO_2$, optische Schichtdicke $\leq$ 150 nm, bevorzugt aus einem Bereich von 40 nm bis 140 nm
Schicht C: Titanoxid, vorzugsweise $TiO_2$
Schicht A: nicht selektiv absorbierendes Eisenoxid, vorzugsweise $Fe_3O_4$
Schicht B: Siliziumoxid, bevorzugt $SiO_2$, optische Schichtdicke $\leq$ 150 nm, bevorzugt aus einem Bereich von 40 nm bis 140 nm
Schicht C: selektiv absorbierendes Eisenoxid, vorzugsweise $Fe_2O_3$, und Titanoxid, vorzugsweise $TiO_2$
Schicht A: nicht selektiv absorbierendes Eisenoxid, vorzugsweise $Fe_3O_4$
Schicht B: Siliziumoxid, bevorzugt $SiO_2$, optische Schichtdicke $\leq$ 150 nm, bevorzugt aus einem Bereich von 40 nm bis 140 nm
Schicht C: nicht selektiv absorbierendes Eisenoxid, vorzugsweise $Fe_3O_4$, und Titanoxid, vorzugsweise $TiO_2$
Schicht A: nicht selektiv absorbierendes Eisenoxid, vorzugsweise $Fe_3O_4$
Schicht B: Siliziumoxid, bevorzugt $SiO_2$, optische Schichtdicke $\leq$ 150 nm, bevorzugt aus einem Bereich von 40 nm bis 140 nm
Schicht C: selektiv absorbierendes Eisenoxid, vorzugsweise $Fe_2O_3$, und nicht selektiv absorbierendes Eisenoxid, vorzugsweise $Fe_3O_4$
Die hochbrechenden Schichten A und C für die vorgenannten bevorzugten Mehrschichtperlglanzpigmente weisen unabhängig voneinander bevorzugt eine optische Schichtdicke von 40 bis 880 nm und weiter bevorzugt 50 bis 850 nm auf.

II. Mehrschichtperlglanzpigmente mit wenigstens zwei Interferenzfarben, d.h. mit winkeiabhängigem Wechsel der Interferenzfarbe

Schicht A: selektiv absorbierendes Eisenoxid, vorzugsweise $Fe_2O_3$
Schicht B: Siliziumoxid, bevorzugt $SiO_2$, optische Schichtdicke >150 nm, bevorzugt 180 bis 480 nm
Schicht C: Titanoxid, vorzugsweise Titandioxid
Schicht A: selektiv absorbierendes Eisenoxid, vorzugsweise $Fe_2O_3$
Schicht B: Siliziumoxid, bevorzugt $SiO_2$, optische Schichtdicke >150 nm, bevorzugt 180 bis 480 nm
Schicht C: Titanoxid, vorzugsweise Titandioxid, und selektiv absorbierendes Eisenoxid, vorzugsweise $Fe_2O_3$,

Schicht A: selektiv absorbierendes Eisenoxid, vorzugsweise $Fe_2O_3$

Schicht B: Siliziumoxid, bevorzugt $SiO_2$, optische Schichtdicke >150 nm, bevorzugt 180 bis 480 nm

Schicht C: Titanoxid, vorzugsweise Titandioxid, und nicht selektiv absorbierendes Eisenoxid, vorzugsweise $Fe_3O_4$

Schicht A: selektiv absorbierendes Eisenoxid, vorzugsweise $Fe_2O_3$

Schicht B: Siliziumoxid, bevorzugt $SiO_2$, optische Schichtdicke >150 nm, bevorzugt 180 bis 480 nm

Schicht C: nicht selektiv absorbierendes Eisenoxid, vorzugsweise $Fe_3O_4$ und selektiv absorbierendes Eisenoxid, vorzugsweise $Fe_2O_3$

Schicht A: selektiv absorbierendes Eisenoxid, vorzugsweise $Fe_2O_3$

Schicht B: Siliziumoxid, bevorzugt $SiO_2$, optische Schichtdicke >150 nm, bevorzugt 180 bis 480 nm

Schicht C: selektiv absorbierendes Eisenoxid, vorzugsweise $Fe_2O_3$

Schicht A: nicht selektiv absorbierendes Eisenoxid, vorzugsweise $Fe_3O_4$

Schicht B: Siliziumoxid, bevorzugt $SiO_2$, optische Schichtdicke >150 nm, bevorzugt 180 bis 480 nm

Schicht C: selektiv absorbierendes Eisenoxid, vorzugsweise $Fe_2O_3$

Schicht A: nicht selektiv absorbierendes Eisenoxid, vorzugsweise $Fe_3O_4$

Schicht B: Siliziumoxid, bevorzugt $SiO_2$, optische Schichtdicke >150 nm, bevorzugt 180 bis 480 nm

Schicht C: Titanoxid, vorzugsweise $TiO_2$

Schicht A: nicht selektiv absorbierendes Eisenoxid, vorzugsweise $Fe_3O_4$

Schicht B: Siliziumoxid, bevorzugt $SiO_2$, optische Schichtdicke >150 nm, bevorzugt 180 bis 480 nm

Schicht C: selektiv absorbierendes Eisenoxid, vorzugsweise $Fe_2O_3$, und Titanoxid, vorzugsweise $TiO_2$,

Schicht A: nicht selektiv absorbierendes Eisenoxid, vorzugsweise $Fe_3O_4$

Schicht B: Siliziumoxid, bevorzugt $SiO_2$, optische Schichtdicke >150 nm, bevorzugt 180 bis 480 nm

Schicht C: nicht selektiv absorbierendes Eisenoxid, vorzugsweise $Fe_3O_4$, und Titanoxid, vorzugsweise $TiO_2$,

Schicht A: nicht selektiv absorbierendes Eisenoxid, vorzugsweise $Fe_3O_4$

Schicht B: Siliziumoxid, bevorzugt $SiO_2$, optische Schichtdicke >150 nm, bevorzugt 180 bis 480 nm

Schicht C: selektiv absorbierendes Eisenoxid, vorzugsweise $Fe_2O_3$, und nicht selektiv absorbierendes Eisenoxid, vorzugsweise $Fe_3O_4$

Die hochbrechenden Schichten A und C für die vorgenannten bevorzugten Mehrschichtperlglanzpigmente weisen unabhängig voneinander bevorzugt eine optische Schichtdicke von 40 bis 880 nm und weiter bevorzugt 50 bis 850 nm auf.

Bei den in dieser Anmeldung angegeben Schichtdicken handelt es sich - sofern nicht anders angegeben - um die optische Schichtdicken. Unter einer optischen Schichtdicke wird erfindungsgemäß das Produkt aus geometrischer Schichtdicke und dem Brechungsindex des die Schicht aufbauenden Materials verstanden. Als Wert für den Brechungsindex des jeweiligen Materials wird der aus der Literatur jeweils bekannte Wert verwendet. Die geometrische Schichtdicke wird dabei erfindungsgemäß anhand von Querschliffaufnahmen von Lacken, in denen planparallel zum Untergrund orientierte Mehrschichtperlglanzpigmente vorliegen, mittels REM bestimmt.

Die Mehrschichtperlglanzpigmente können weiterhin mit wenigstens einer äußeren Schutzschicht D versehen sein, die die Licht-, Wetter-, und/ oder chemische Stabilität des Mehrschichtperlglanzpigmentes weiter erhöht. Bei der äußeren Schutzschicht D kann es sich auch um eine Nachbeschichtung handeln, die die Handhabung des Pigments bei Einarbeitung in unterschiedliche Medien erleichtert.

Die äußere Schutzschicht D der erfindungsgemäßen Mehrschichtperlglanzpigmente kann eine oder zwei Metalloxidschichten der Elemente Si, Al oder Ce umfassen bzw. bevorzugt daraus bestehen. Bei einer Variante wird als äußerste Metalloxidschicht eine Siliziumoxidschicht, vorzugsweise $SiO_2$-Schicht, aufgebracht. Besonders bevorzugt ist hierbei eine Reihenfolge, bei der zunächst eine Ceroxidschicht aufgebracht ist, der dann eine $SiO_2$-Schicht folgt, wie in der WO 2006/021386 A1 beschrieben, deren Inhalt hiermit unter Bezugnahme aufgenommen ist.

Die äußere Schutzschicht D kann des Weiteren an der Oberfläche organischchemisch modifiziert sein. Beispielsweise können ein oder mehrere Silane auf dieser äußeren Schutzschicht aufgebracht sein. Bei den Silanen kann es sich um Alkylsilane mit verzweigtkettigen oder unverzweigten Alkylresten mit 1 bis 24 C-Atomen, vorzugsweise 6 bis 18 C-Atomen handeln.

Bei den Silanen kann es sich aber auch um organofunktionelle Silane handeln, die eine chemische Anbindung an einen Kunststoff, ein Bindemittel eines Lackes oder einer Farbe, etc. ermöglichen.

Die vorzugsweise als Oberflächenmodifizierungsmittel verwendeten organofunktionellen Silane, die geeignete funktionelle Gruppen aufweisen, sind kommerziell verfügbar und werden beispielsweise von der Fa. Evonik hergestellt und unter dem Handelsnamen "Dynasylan" vertrieben. Weitere Produkte können von der Fa. Momentive (Silquest-Silane) oder von der Fa. Wacker, beispielsweise Standard- und $\alpha$-Silane aus der GENIOSIL-Produktgruppe, bezogen werden. Beispiele hierfür sind 3-Methacryloxypropyltrimethoxysilan (Dynasylan MEMO, Silquest A-174NT), Vinyltri(m)ethoxysilan (Dynasylan VTMO bzw. VTEO, Silquest A-151 bzw. A-171). Methyltri(m)ethoxysilan (Dynasylan MTMS bzw. MTES), 3-Mercaptopropyltrimethoxysilan (Dynasylan MTMO; Silquest A-189), 3-Glycidoxypropyltrimethoxysilan (Dynasylan GLYMO, Silquest A-187), Tris[3-(trimethoxysilyl)propyl]isocyanurat (Silquest Y-11597), Bis[3-(triethoxysilyl)propyl)]tetrasulfid (Silquest A-1289), Bis[3-(triethoxysilyl)propyldisulfid (Silquest A-1589), beta-(3,4-Epoxycyclohexyl) ethyltrimethoxysilan (Silquest A-186), Bis(triethoxysilyl)ethan (Silquest Y-9805), gamma-Isocyanatopropyltrimethoxysilan (Silquest A-Link 35, GENIOSIL GF40), Methacryloxymethyltri(m)ethoxysilan (GENIOSIL XL 33, XL 36), (Methacryloxymethyl)(m)ethyldimethoxysilan (GENIOSIL XL 32, XL 34), (Isocyanatomethyl)methyldimethoxysilan, (Isocyanatomethyl)trimethoxysilan, 3-(Triethoxysilyl)propylbernsteinsäureanhydrid (GENIOSIL GF 20), (Methacryloxymethyl)methyldiethoxysilan, 2-Acryloxyethylmethyldimethoxysilan, 2-Methacryloxyethyltrimethoxysilan, 3-Acryloxypropylmethyldimethoxysilan, 2-Acryloxyethyltrimethoxysilan, 2-Methacrytoxyethyltriethoxysilan, 3-Acryloxypropyltrimethoxysilan, 3-Acryloxypropyltripropoxysilan, 3-Methacryloxypropyltriethoxysilan, 3-Methacryloxypropyltriacetoxysilan, 3- Methacryloxypropylmethyldimethoxysilan, Vinyltrichlorsilan, Vinyltrimethoxysilan (GENIOSIL XL 10), Vinyltris(2-methoxyethoxy)silan (GENIOSIL GF 58), Vinyltriacetoxysilan.

Vorzugsweise werden als organofunktionelle Silane 3-Methacryloxypropyltrimethoxysilan (Dynasylan MEMO, Silquest A-174NT), Vinyltri(m)ethoxysitan (Dynasylan VTMO bzw. VTEO, Silquest A-151 bzw. A-171), Methyltri(m) ethoxysilan (Dynasylan MTMS bzw. MTES), beta-(3,4-Epoxycyclohexyl)ethyltrimethoxysilan (Silquest A-186), Bis (triethoxysilyl)ethan (Silquest Y-9805), gamma-Isocyanatopropyltrimethoxysilan (Silquest A-Link 35, GENIOSIL GF40), Methacryloxymethyltri(m)ethoxysilan (GENIOSIL XL 33, XL 36), (Methacryloxymethyl)(m)ethyldimethoxysilan (GENIOSIL XL 32, XL 34), 3-(Triethoxysilyl)propylbernsteinsäureanhydrid (GENIOSIL GF 20), Vinyltrimethoxysilan (GENIOSIL XL 10) und/ oder Vinyltris(2-methoxyethoxy)silan (GENIOSIL GF 58) verwendet.

Es ist aber auch möglich, andere organofunktionelle Silane auf die erfindungsgemäßen Mehrschichtperlglanzpigmente aufzubringen.

Weiterhin lassen sich, beispielsweise kommerziell von Degussa erhältliche, wässrige Vorhydrolysate einsetzen. Hierzu gehören u.a. wässriges Aminosiloxan (Dynasylan Hydrosil 1151), wässriges amino-/ alkylfunktionelles Siloxan (Dynasylan Hydrosil 2627 oder 2909), wässriges diaminofunktionelles Siloxan (Dynasylan Hydrosil 2776), wässriges epoxyfunktionelles Siloxan (Dynasylan Hydrosil 2926), amino-/ alkylfunktionelles Oligosiloxan (Dynasylan 1146), vinyl-/ alkylfunktionelles Oligosiloxan (Dynasylan 6598), oligomeres Vinylsilan (Dynasylan 6490) oder oligomeres kurzkettiges alkylfunktionelles Silan (Dynasylan 9896).

Bei einer bevorzugten Ausführungsform enthält das organofunktionelle Silangemisch neben wenigstens einem Silan ohne funktionelle Bindungsgruppe wenigstens ein aminofunktionelles Silan. Die Aminofunktion ist eine funktionelle Gruppe, die mit den meisten in Bindemitteln vorhandenen Gruppen eine oder mehrere chemische Wechselwirkungen eingehen kann. Dies kann eine kovalente Bindung, wie z.B. mit Isocyanat- oder Carboxylatfunktionen des Bindemittels, oder Wasserstoffbrückenbindungen wie mit OH- oder COOR-Funktionen oder auch ionische Wechselwirkungen beinhalten. Eine Aminofunktion ist daher für den Zweck der chemischen Anbindung des Mehrschichtperlglanzpigments an verschiedenartige Bindemittel sehr gut geeignet.

Bevorzugt werden hierzu folgende Verbindungen genommen: 3-Aminopropyltrimethoxysilan (Dynasylan AMMO; Silquest A-1110), 3-Aminopropyltriethoxysilan (Dynasylan AMEO), [3-(2-Aminoethyl)aminopropyl]trimethoxysilan (Dynasylan DAMO, Silquest A-1120), [3-(2-Aminoethyl)-aminopropyl]triethoxysilan, triaminofunktionelles Trimethoxysilan (Silquest A-1130), Bis-(gamma-trimethoxysilylpropyl)amin (Silquest A-1170), N-Ethyl-gammaaminoisobutyltrimethoxysilan (Silquest A-Link 15), N-Phenyl-gamma-aminopropyltrimethoxysilan (Silquest Y-9669), 4-Amino-3,3-dimethylbutyltrimethoxysilan (Silquest A-1637), N-Cyclohexylaminomethylmethyldiethoxysilan (GENIOSIL XL 924), N-Cyclohexylaminomethyltriethoxysilan (GENIOSIL XL 926), N-Phenylaminomethyltrimethoxysilan (GENIOSIL XL 973) und deren Mischungen.

Bei einer weiterhin bevorzugten Ausführungsform ist das Silan ohne funktionelle Bindungsgruppe ein Alkylsilan. Das Alkylsilan weist vorzugsweise die Formel (A) auf:

$$R_{(4-z)}Si(X)_z, \qquad (A)$$

Hierbei ist z eine ganze Zahl von 1 bis 3, R ist eine substituierte oder unsubstituierte, unverzweigte oder verzweigte Alkylkette mit 10 bis 22 C-Atomen und X steht für eine Halogen- und/ oder Alkoxygruppe. Bevorzugt sind Alkylsilane mit Alkylketten mit mindestens 12 C-Atomen. R kann auch zyklisch mit Si verbunden sein, wobei in diesem Fall z üblicherweise 2 ist.

An oder auf der Oberfläche der erfindungsgemäßen Mehrschichtperlglanzpigmente können neben den erwähnten Silanen bzw. Sitangemischen auch weitere organisch-chemische Modifizierungsmittel, wie beispielsweise substituierte oder nicht substituierte Alkylreste, Polyether, Thioether, Siloxane, etc. und Mischungen davon angeordnet sein. Es können aber auch anorganisch-chemische Modifizierungsmittel (z. B. $Al_2O_3$ oder $ZrO_2$ oder deren Gemische), welche z. B. die Dispergierbarkeit und/ oder Verträglichkeit im jeweiligen Anwendungsmedium erhöhen können, auf die Pigmentoberfläche aufgebracht werden.

Über die Oberflächenmodifizierung kann beispielsweise die Hydrophilie oder Hydrophobie der Pigmentoberfläche verändert und/ oder eingestellt werden. Beispielsweise können über die Oberflächenmodifizierung die Leafingbzw. Nonleafing-Eigenschaften der erfindungsgemäßen Mehrschichtperlglanzpigmente verändert und/ oder eingestellt werden. Unter Leafing wird verstanden, dass sich die erfindungsgemäßen Mehrschichtperlglanzpigmente in einem Anwendungsmedium, beispielsweise einem Lack oder einer Druckfarbe, an oder in der Nähe der Grenz- oder Oberfläche des Anwendungsmediums anordnen.

Die Oberflächenmodifizierungsmittel können auch reaktive chemische Gruppen, wie beispielsweise Acrylat-, Methacrylat-, Vinyl-, Isocyanat-, Cyano-, Epoxy-, Hydroxy-, Aminogruppen oder Mischungen davon, aufweisen. Diese chemisch reaktiven Gruppen ermöglichen eine chemische Anbindung, insbesondere Ausbildung von kovalenten Bindungen, an das Anwendungsmedium oder Komponenten des Anwendungsmediums, wie beispielsweise Bindemittel. Hierdurch können beispielsweise die chemischen und/ oder physikalischen Eigenschaften von ausgehärteten Lacken, Farben oder Druckfarben wie Beständigkeit gegenüber Umwelteinflüssen wie Feuchtigkeit, Sonneneinstrahlung, UV-Beständigkeit, etc., oder gegenüber mechanischen Einflüssen, beispielsweise Kratzern etc., verbessert werden.

Die chemische Reaktion zwischen den chemisch-reaktiven Gruppen und dem Anwendungsmedium bzw. Komponenten des Anwendungsmediums kann beispielsweise durch Einstrahlung von Energie, beispielsweise in Form von UV-Strahlung und/ oder Wärme, induziert werden.

Für die Einarbeitung von mit Silanen nachbeschichteten bzw. mit einer äußeren Schutzschicht versehenen Mehrschichtperlglanzpigmenten in kosmetischen Formulierungen muss darauf geachtet werden, dass das entsprechende Silan bzw. das Material der äußeren Schutzschicht nach der Kosmetikverordnung zulässig ist.

Die erfindungsgemäßen Mehrschichtperlglanzpigmente eignen sich insbesondere für die Anwendung in Kosmetika, wie z.B. Körperpuder, Gesichtspuder, gepresstem und losem Puder, Gesichtsmakeup, Pudercreme, Crememakeup, Emulsionsmakeup, Wachsmakeup, Foundation, Moussemakeup, Wangenrouge, Augenmakeup wie Lidschatten, Mascara, Eyeliner, flüssige Eyeliner, Augenbrauenstift, Lippenpflegestift, Lippenstift, Lip Gloss, Lip Liner, Haarstylingkompositionen wie Haarspray, Haarmousse, Haargel, Haarwachs, Haarmascara, permanente oder semipermanente Haarfarben, temporäre Haarfarben, Hautpflegekompositionen wie Lotions, Gele, Emulsionen sowie Nagellackkompositionen.

Zur Erzielung spezieller Farbeffekte können in den Kosmetikapplikationen neben den erfindungsgemäßen Mehrschichtperlglanzpigmenten weitere Farbmittel und/ oder herkömmliche Effektpigmente oder Mischungen hiervon in variablen Mengenverhältnissen eingesetzt werden. Als herkömmliche Effektpigmente können beispielsweise handelsübliche Perlglanzpigmente auf der Basis von mit hochbrechenden Metalloxiden beschichtetem natürlichen Glimmer (wie z.B. die Produktgruppe Prestige der Fa. Eckart), BiOCl-Plättchen, $TiO_2$-Plättchen, Perlglanzpigmente auf der Basis von mit hochbrechenden Metalloxiden beschichtetem synthetischen Glimmer oder basierend auf mit hochbrechenden Metalloxiden beschichteten Glasplättchen (wie z.B. die Produktgruppe MIRAGE der Fa. Eckart), $Al_2O_3$-, $SiO_2$- oder $TiO_2$-Plättchen verwendet werden. Außerdem können auch Metalleffektpigmente, wie z.B. die Produktgruppe Visionaire der Fa. Eckart, zugegeben werden. Die Farbmittel können aus anorganischen oder organischen Pigmenten ausgewählt sein.

Ein Verfahren zur Herstellung der erfindungsgemäßen Mehrschichtperlglanzpigmente umfasst die folgenden Schritte:

i) Größenklassieren der zu beschichtenden plättchenförmigen transparenten Substrate, so dass die zu beschichtenden plättchenförmigen transparenten Substrate eine volumengemittelte Größenverteilungsfunktion mit den Kennzahlen $D_{10}$, $D_{50}$, $D_{90}$ und einem Span $\Delta D$ in einem Bereich von 0,7 -1,4 aufweisen, wobei der Span $\Delta D$ gemäß der Formel $\Delta D = (D_{90} - D_{10})/ D_{50}$ definiert ist,
ii) Aufbringen wenigstens der Schichten A bis C auf die plättchenförmigen transparenten Substrate sowie optional wenigstens einer Schicht D,
oder
iii) Aufbringen wenigstens der Schichten A bis C auf die plättchenförmigen transparenten Substrate sowie optional wenigstens einer Schicht D,
iv) Größenklassieren der zu beschichtenden plättchenförmigen transparenten Substrate, so dass die zu beschichtenden plättchenförmigen transparenten Substrate eine volumengemittelte Größenverteilungsfunktion mit den Kennzahlen $D_{10}$, $D_{50}$, $D_{90}$ und einen Span $\Delta D$ in einem Bereich von 0,7 - 1,4 aufweisen, wobei der Span $\Delta D$ gemäß der Formel $\Delta D = (D_{90} - D_{10})/ D_{50}$ definiert ist.

Wenn die Ausgangssubstrate zu groß sind, kann optional ein Zerkleinerungsschritt vor dem Größenklassieren durchgeführt werden.

Die Größenklassierung kann vor oder nach der Beschichtung der Substrate erfolgen. Vorteilhafterweise wird jedoch zuerst das Substrat klassiert und anschließend beschichtet. Die Größenklassierung wird durchgeführt und gegebenenfalls wiederholt, bis die Mehrschichtperlglanzpigmente die erfindungsgemäße Größenverteilung aufweisen.

Ein enger Span $\Delta D$ der Substrate kann durch geeignete Zerkleinerungs- und/ oder Klassierungsprozesse der zu beschichtenden plättchenförmigen transparenten Substrate erreicht werden. Die zu beschichtenden plättchenförmigen transparenten Substrate können beispielsweise durch Kugelmühle, Strahl- oder Rührwerkskugelmühle, Kollergang oder Dissolver zerkleinert werden. Der Span ΔD der Endfraktion kann durch geeignete Klassierung, wie beispielsweise eine mehrfache Nasssiebung, eingestellt werden. Weitere Klassierungsverfahren umfassen die Zentrifugation in Zyklonen oder eine Sedimentation aus einer Dispersion.

Die Zerkleinerungs- und Klassierprozesse können nacheinander erfolgen und gegebenenfalls miteinander kombiniert werden. So kann auf einen Zerkleinerungsvorgang ein Klassierungsvorgang folgen, dem sich ein weiterer Zerkleinerungsvorgang des Feinguts anschließt usw.

Die Metalloxidschichten werden vorzugsweise nasschemisch aufgebracht, wobei die zur Herstellung von Perlglanzpigmenten entwickelten nasschemischen Beschichtungsverfahren angewendet werden können. Bei der Nassbeschichtung werden die Substratpartikel in Wasser suspendiert und mit einem oder mehreren hydrolysierbaren Metallsalzen oder einer Wasserglaslösung bei einem für die Hydrolyse geeigneten pH-Wert versetzt, der so gewählt wird, dass die Metalloxide bzw. Metalloxidhydrate direkt auf dem zu beschichtenden Substrat aufgefällt werden, ohne dass es zu Nebenfällungen kommt. Der pH-Wert wird üblicherweise durch gleichzeitiges Zudosieren einer Base und/ oder einer Säure konstant gehalten. Anschließend werden die Pigmente abgetrennt, gewaschen und bei 50 -150°C für 6 -18 Stunden getrocknet und gegebenenfalls 0,5 - 3 Stunden geglüht, wobei die Glühtemperatur im Hinblick auf die jeweils vorliegende Beschichtung optimiert werden kann. In der Regel liegen die Glühtemperaturen zwischen 500 und 1000°C, vorzugsweise zwischen 600 und 900°C. Falls gewünscht können die Pigmente nach Aufbringen einzelner Beschichtungen abgetrennt, getrocknet und gegebenenfalls geglüht werden, um dann zur Auffällung der weiteren Schichten wieder resuspendiert zu werden.

Die Auffällung der $SiO_2$-Schicht auf das zu beschichtende plättchenförmige transparente Substrat kann durch Zugabe einer Kalium- oder Natronwasserglaslösung bei einem geeigneten pH-Wert erfolgen. Alternativ kann die $SiO_2$-Schicht über Sol-Gel-Verfahren ausgehend von Alkoxysilanen, wie z.B. Tetraethoxysilan, aufgebracht werden. Im Folgenden wird die Erfindung durch einige Beispiele näher erläutert, ohne auf diese beschränkt zu sein.

**I Herstellung der Pigmente**

**A Klassierung der Substrate**

**Beispiel 1: Klassierung von Glasflakes mit engem Span ΔD = 1,0**

Eine Suspension von 200 g Glasflakes (GF100M der Firma Glassflake Ltd.) in VE-Wasser (VE = vollentsatzt, ca. 3 Gew.-%ig) wurde über ein 100 μm Sieb klassiert und der Siebdurchgang wiederum über ein 63 μm Sieb gesiebt. Diese Siebprozedur wurde mit auf dem 63 μm Sieb erhaltenen Siebrückstand zweimal wiederholt. Es wurde eine Glasflakefraktion erhalten, die folgende Partikelgrößenverteilung aufwies (MALVERN Mastersizer 2000): $D_{10}$=50 μm, $D_{50}$=82 μm, $D_{90}$=132 μm, Span ΔD = 1,0.

**Vergleichsbeispiel 1: Klassierung von Glasflakes mit breitem Span ΔD = 2,0**

Eine Suspension von 200 g Glasflakes (GF100M der Firma Glassflake Ltd.) in VE-Wasser (ca. 3 Gew.-%ig) wurde über ein 150 μm Sieb klassiert und der Siebdurchgang wiederum über ein 34 μm Sieb gesiebt. Diese Siebprozedur wurde mit auf dem 34 μm Sieb erhaltenen Siebrückstand zweimal wiederholt. Es wurde eine Glasflakefraktion erhalten, die folgende Partikelgrößenverteilung aufwies (MALVERN Mastersizer 2000): $D_{10}$=25 μm, $D_{50}$=88 μm, $D_{90}$=200 μm, Span ΔD = 1,99.

**Beispiel 2: Klassierung von synthetischem Glimmer mit engem Span ΔD =1,2**

Eine Suspension von 200 g Artificial Mica Sanbao 10-40 (Fa. Shantou F.T.Z. Sanbao Pearl Luster Mica Tech Co., Ltd. China) in VE-Wasser (ca. 3 Gew.-%ig) wurde über ein 34 μm Sieb klassiert und der Siebdurchgang wiederum über ein 20 μm Sieb gesiebt. Diese Siebprozedur wurde mit auf dem 20 μm Sieb erhaltenen Siebrückstand zweimal wiederholt. Es wurde eine Glimmerfraktion erhalten, die folgende Partikelgrößenverteilung aufwies (MALVERN Mastersizer 2000): $D_{10}$=14 μm. $D_{50}$=26 μm, $D_{90}$=45 μm, Span ΔD = 1,2.

**Vergleichsbeispiel 2: Klassierung von synthetischem Glimmer mit breitem Span ΔD = 3,7**

1000g handelsüblicher unklassierter synthetischer Mica Artificial Mica Sanbao unclassified (Fa. Shantou F.T.Z. Sanbao Pearl Luster Mica Tech Co., Ltd. China) wurde mit 1000 ml VE-Wasser versetzt und dann in einem Laborkoller der Fa. American Cyanamid Company ca. 1 h delaminiert.

Der Kuchen wurde anschließend mit VE-Wasser zu einem Feststoffgehalt von 25 Gew.-% verdünnt und in einem Labordissolver der Fa. Pendraulik des Typs TD 200 1h behandelt. Hierbei ist darauf zu achten, dass durch Kühlung die Temperatur der Suspension 80°C nicht übersteigt.

Anschließend wurde die Suspension mit VE-Wasser auf 3 Gew.-% Feststoffgehalt gebracht und über ein Laborsieb des Typs Separator der Fa. Sweco < 250 μm gesiebt.

Die so erhaltene Micafraktion wurde dann über einen Büchner Trichter abgesaugt und der erhaltene Filterkuchen als Ausgangsmaterial für weitere Beschichtungen verwendet.

Es wurde eine Glimmerfraktion erhalten, die folgende Partikelgrößenverteilung aufwies (MALVERN Mastersizer 2000): $D_{10}$= 17,7 μm, $D_{50}$= 74,6 μm, $D_{90}$= 292,3 μm, Span $\Delta D$ = 3,7.

**B Herstellung einschichtiger Pigmente (Ausgangsmaterial für mehrschichtige Perlglanzpigmente)**

**Vergleichsbeispiel 3: Herstellung des Ausgangsmaterials für die Beispiele 3 und 4**

200 g Glasflakes aus Beispiel 1 wurden in 2000 ml VE-Wasser suspendiert und unter turbulentem Rühren auf 80°C erhitzt. Der pH-Wert der Suspension wurde mit verdünnter HCl auf pH 1,9 eingestellt und sodann eine erste Schicht "$SnO_2$" auf den Glasflakes gefällt. Diese Schicht wurde durch Zugabe von einer Lösung, bestehend aus 3 g $SnCl_4$ x 5 $H_2O$ (in 10 ml konz. HCl plus 50 ml VE-Wasser), unter gleichzeitiger Zudosierung von einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 3,0 angehoben und sodann eine Lösung von 42 ml $FeCl_3$ (280 g $Fe_2O_3$/l) in die Suspension hinzudosiert. Der pH-Wert wurde dabei durch Gegensteuern mit 10 Gew.-%iger NaOH-Lösung konstant auf pH 3,0 gehalten. Danach wurde noch 15 min nachgerührt, abfiltriert und der Filterkuchen mit VE-Wasser nachgewaschen. Der Filterkuchen wurde bei 100°C vorgetrocknet und bei 650°C 30 min kalziniert. Es wurde ein glänzendes Effektpigment mit silberner Interferenzfarbe und leicht orange-roter Absorptionsfarbe erhalten.

**Vergleichsbeispiel 4 : Herstellung des Ausgangsmaterials für die Vergleichsbeispiele 6 und 7**

200 g Glasflakes aus Vergleichsbeispiel 1 wurden in 2000 ml VE-Wasser suspendiert und unter turbulentem Rühren auf 80°C erhitzt. Der pH-Wert der Suspension wurde mit verdünnter HCl auf pH 1,9 eingestellt und sodann eine erste Schicht "$SnO_2$" auf den Glasflakes gefällt. Diese Schicht wurde durch Zugabe von einer Lösung, bestehend aus 3 g $SnCl_4$ x 5 $H_2O$ (in 10 ml konz. HCl plus 50 ml VE-Wasser), unter gleichzeitiger Zudosierung von einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 3,0 angehoben und sodann eine Lösung von 42 ml $FeCl_3$ (280 g $Fe_2O_3$/l) in die Suspension hinzudosiert. Danach wurde noch 15 min nachgerührt, abfiltriert und der Filterkuchen mit VE-Wasser nachgewaschen. Der Filterkuchen wurde bei 100°C vorgetrocknet und bei 650°C 30 min kalziniert. Es wurde ein Effektpigment mit silberner Interferenzfarbe und leicht orange-roter Absorptionsfarbe erhalten.

**Vergleichsbeispiel 5: synthetischer Glimmer/ $Fe_2O_3$**

200 g synthetischer Glimmer aus Beispiel 2 wurden in 2000 ml VE-Wasser suspendiert und unter turbulentem Rühren auf 80°C erhitzt. Der pH-Wert der Suspension wurde mit verdünnter HCl auf pH 3,0 eingestellt und sodann eine Lösung von 230 ml $FeCl_3$ (280 g $Fe_2O_3$/l VE-Wasser) in die Suspension hinzudosiert. Der pH-Wert wurde dabei durch Gegensteuern mit 10 Gew.-%iger NaOH-Lösung konstant auf pH 3,0 gehalten. Danach wurde noch 15 min nachgerührt, abfiltriert und der Filterkuchen mit VE-Wasser nachgewaschen. Der Filterkuchen wurde bei 100°C vorgetrocknet und bei 750°C 30 min kalziniert. Es wurde ein glänzendes bronzefarbenes Perlglanzpigment erhalten.

**C Herstellung der Mehrschichtperlglanzpigmente**

**Beispiel 3: Glasflakes/ $Fe_2O_3$/ $SiO_2$/ $Fe_2O_3$**

200 g Glasflakes aus Vergleichsbeispiel 3 wurden in 1400 ml Isopropanol suspendiert und unter turbulentem Rühren auf 70°C erhitzt. Zu dieser Suspension gab man 75 g Tetraethoxysilan, 75 g VE-Wasser und 5 ml 10 Gew.-%ige $NH_3$-Lösung. Die Reaktionsmischung wurde für ca. 12 h gerührt, danach abfiltriert, der Filterkuchen mit Isopropanol nachgewaschen und im Vakuumtrockenschrank bei 100°C getrocknet.

100 g der so erhaltenen $SiO_2$-beschichteten Glasflakes wurden in 700 ml VE-Wasser suspendiert und unter turbulentem Rühren auf 80°C erhitzt. Der pH-Wert der Suspension wurde mit verdünnter HCl auf pH 1,9 eingestellt und sodann eine erste Schicht "$SnO_2$" auf den beschichteten Glasflakes gefällt. Diese Schicht wurde durch Zugabe von einer Lösung, bestehend aus 1.5 g $SnCl_4$ x 5 $H_2O$ (in 5 ml konz. HCl plus 25 ml VE-Wasser), unter gleichzeitiger Zudosierung von einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 3,0 angehoben und sodann eine Lösung von 42,5 ml FeCl$_3$ (280 g Fe$_2$O$_3$/l) in die Suspension hinzudosiert. Danach wurde noch 15 min nachgerührt, abfiltriert und der Filterkuchen mit VE-Wasser nachgewaschen. Der Filterkuchen wurde bei 100°C vorgetrocknet und bei 650°C 30 min kalziniert. Es wurde ein stark glänzendes Mehrschichtperlglanzpigment mit oranger Interferenzfarbe und roter Absorptionsfarbe erhalten.

**Beispiel 4: Glasflakes/ Fe$_2$O$_3$/ SiO$_2$/ TiO$_2$(rutil)**

200 g Glasflakes aus Vergleichsbeispiel 3 wurden in 1400 ml Isopropanol suspendiert und unter turbulentem Rühren auf 70°C erhitzt. Zu dieser Suspension gab man 75 g Tetraethoxysilan, 75 g VE-Wasser und 5 ml 10 Gew.-%ige NH$_3$-Lösung. Die Reaktionsmischung wurde für ca. 12 h gerührt, danach abfiltriert, der Filterkuchen mit Isopropanol nachgewaschen und im Vakuumtrockenschrank bei 100°C getrocknet.
100 g der so erhaltenen SiO$_2$-beschichteten Glasflakes wurden in 700 ml VE-Wasser suspendiert und unter turbulentem Rühren auf 80°C erhitzt. Der pH-Wert der Suspension wurde mit verdünnter HCl auf pH 1,9 eingestellt und sodann eine erste Schicht "SnO$_2$" auf den beschichteten Glasflakes gefällt. Diese Schicht wurde durch Zugabe von einer Lösung, bestehend aus 1.5 g SnCl$_4$ x 5 H$_2$O (in 5 ml konz. HCl plus 25 ml VE-Wasser), unter gleichzeitiger Zudosierung von einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 1,6 heruntergesetzt und sodann eine Lösung von 85 ml TiCl$_4$ (200 g TiO$_2$/l VE-Wasser) in die Suspension hinzudosiert. Der pH-Wert wurde dabei durch Gegensteuern mit 10 Gew.-%iger NaOH-Lösung konstant auf pH 1,6 gehalten. Danach wurde noch 15 min nachgerührt, abfiltriert und der Filterkuchen mit VE-Wasser nachgewaschen. Der Filterkuchen wurde bei 100°C vorgetrocknet und bei 650°C 30 min kalziniert. Es wurde ein extrem hochglänzendes Effektpigment mit grüner Interferenzfarbe erhalten.

**Vergleichsbeispiel 6: Glasflakes/ Fe$_2$O$_3$/ SiO$_2$/ Fe$_2$O$_3$**

200 g Glasflakes aus Vergleichsbeispiel 4 wurden in 1400 ml Isopropanol suspendiert und unter turbulentem Rühren auf 70°C erhitzt. Zu dieser Suspension gab man 75 g Tetraethoxysilan, 75 g VE-Wasser und 5 ml 10 Gew.-%ige NH$_3$-Lösung. Die Reaktionsmischung wurde für ca. 12 h gerührt, danach abfiltriert, der Filterkuchen mit Isopropanol nachgewaschen und im Vakuumtrockenschrank bei 100°C getrocknet.
100 g der so erhaltenen SiO$_2$-beschichteten Glasflakes wurden in 700 ml VE-Wasser suspendiert und unter turbulentem Rühren auf 80°C erhitzt. Der pH-Wert der Suspension wurde mit verdünnter HCl auf pH 1,9 eingestellt und sodann eine erste Schicht "SnO$_2$" auf den beschichteten Glasflakes gefällt. Diese Schicht wurde durch Zugabe von einer Lösung, bestehend aus 1.5 g SnCl$_4$ x 5 H$_2$O (in 5 mi konz. HCl plus 25 ml VE-Wasser), unter gleichzeitiger Zudosierung von einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 3,0 angehoben und sodann eine Lösung von 42.5 ml FeCl$_3$ (280 g Fe$_2$O$_3$/l) in die Suspension hinzudosiert. Danach wurde noch 15 min nachgerührt, abfiltriert und der Filterkuchen mit VE-Wasser nachgewaschen. Der Filterkuchen wurde bei 100°C vorgetrocknet und bei 650°C 30 min kalziniert. Es wurde ein Mehrschichtperlglanzpigment mit rötlich-oranger Interferenzfarbe und roter Absorptionsfarbe erhalten.

**Vergleichsbeispiel 7: Glasflakes/ Fe$_2$O$_3$/ SiO$_2$/ TiO$_2$(rutil)**

200 g Glasflakes aus Vergleichsbeispiel 4 wurden in 1400 ml Isopropanol suspendiert und unter turbulentem Rühren auf 70°C erhitzt. Zu dieser Suspension gab man 75 g Tetraethoxysilan, 75 g VE-Wasser und 5 ml 10 Gew.-%ige NH$_3$-Lösung. Die Reaktionsmischung wurde für ca. 12 h gerührt, danach abfiltriert, der Filterkuchen mit Isopropanol nachgewaschen und im Vakuumtrockenschrank bei 100°C getrocknet.
100 g der so erhaltenen SiO$_2$-beschichteten Glasflakes wurden in 700 ml VE-Wasser suspendiert und unter turbulentem Rühren auf 80°C erhitzt. Der pH-Wert der Suspension wurde mit verdünnter HCl auf pH 1,9 eingestellt und sodann eine erste Schicht "SnO$_2$" auf den beschichteten Glasflakes gefällt. Diese Schicht wurde durch Zugabe von einer Lösung, bestehend aus 1.5 g SnCl$_4$ x 5 H$_2$O (in 5 ml konz. HCl plus 25 ml VE-Wasser), unter gleichzeitiger Zudosierung von einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 1,6 heruntergesetzt und sodann eine Lösung von 85 ml TiCl$_4$ (200 g TiO$_2$/l VE-Wasser) in die Suspension hinzudosiert. Der pH-Wert wurde dabei durch Gegensteuern mit 10 Gew.-%iger NaOH-Lösung konstant auf pH 1,6 gehalten. Danach wurde noch 15 min nachgerührt, abfiltriert und der Filterkuchen mit VE-Wasser nachgewaschen. Der Filterkuchen wurde bei 100°C vorgetrocknet und bei 650°C 30 min kalziniert. Es wurde ein glänzendes Effektpigment mit bläulich-grüner Interferenzfarbe erhalten.

**Beispiel 5: Synthetischer Glimmer/ Fe$_2$O$_3$/ SiO$_2$/ Fe$_2$O$_3$**

200 g synthetischer Glimmer aus Beispiel 2 wurden in 2000 ml VE-Wasser suspendiert und unter turbulentem Rühren auf 80°C erhitzt. Der pH-Wert der Suspension wurde mit verdünnter HCl auf pH 3,0 eingestellt und sodann eine Lösung von 60 ml FeCl$_3$ (280 g Fe$_2$O$_3$/l VE-Wasser) in die Suspension hinzudosiert. Der pH-Wert wurde dabei durch Gegensteuern mit 10 Gew.-%iger NaOH-Lösung konstant auf pH 3,0 gehalten. Um die Fällung zu vervollständigen wurde die Suspension noch weitere 15 min gerührt. Danach wurde der pH-Wert mit 5 Gew.-%iger NaOH-Lösung auf 7,5 angehoben und 15 min gerührt. Eine Wasserglaslösung (185 g Wasserglaslösung, 24 Gew.-% SiO$_2$, gemischt mit 207 g VE-Wasser) wurde sodann langsam in die Suspension eingeleitet und der pH-Wert konstant bei pH 7,5 gehalten. Danach wurde noch 20 min nachgerührt und der pH-Wert wieder auf 3,0 gesenkt und sodann eine Lösung von 200 ml FeCl$_3$ (280 g Fe$_2$O$_3$/l VE-Wasser) in die Suspension hinzudosiert. Der pH-Wert wurde dabei durch Gegensteuern mit 10 Gew.-%iger NaOH-Lösung konstant auf pH 3,0 gehalten. Danach wurde noch 15 min nachgerührt, abfiltriert und der Filterkuchen mit VE-Wasser nachgewaschen. Der Filterkuchen wurde bei 100 C vorgetrocknet und bei 750°C 30 min kalziniert. Es wurde ein sehr brillantes, farbstarkes, bronzefarbenes Mehrschichtperlglanzpigment erhalten.

**Beispiel 6: Synthetischer Glimmer/ Fe$_2$O$_3$/ SiO$_2$/ TiO$_2$(rutil)**

200 g synthetischer Glimmer aus Beispiel 2 wurden in 2000 ml VE-Wasser suspendiert und unter turbulentem Rühren auf 80°C erhitzt. Der pH-Wert der Suspension wurde mit verdünnter HCl auf pH 3,0 eingestellt und sodann eine Lösung von 60 ml FeCl$_3$ (280 g Fe$_2$O$_3$/l VE-Wasser) in die Suspension hinzudosiert. Der pH-Wert wurde dabei durch Gegensteuern mit 10 Gew.-%iger NaOH-Lösung konstant auf pH 3,0 gehalten. Um die Fällung zu vervollständigen wurde die Suspension noch weitere 15 min gerührt. Danach wurde der pH-Wert mit 5 Gew.-%iger NaOH-Lösung auf 7,5 angehoben und 15 min gerührt. Eine Wasserglaslösung (185 g Wasserglaslösung, 24 Gew.-% SiO$_2$, gemischt mit 207 g VE-Wasser) wurde sodann langsam in die Suspension eingeleitet und der pH-Wert konstant bei pH 7,5 gehalten. Danach wurde noch 20 min nachgerührt und der pH-Wert wieder auf 1,9 gesenkt. Dann schied man eine Schicht "SnO$_2$" auf der SiO$_2$-Oberfläche ab. Diese Schicht wurde durch Zugabe von einer Lösung, bestehend aus 5 g SnCl$_4$ x 5 H$_2$O (in 10 ml konz. HCl plus 50 ml VE-Wasser), unter gleichzeitiger Zudosierung von einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 1,6 heruntergesetzt und sodann eine Lösung von 280 ml TiCl$_4$ (200 g TiO$_2$/l VE-Wasser) in die Suspension hinzudosiert. Der pH-Wert wurde dabei durch Gegensteuern mit 10 Gew.-%iger NaOH-Lösung konstant auf pH 1,6 gehalten. Danach wurde noch 15 min nachgerührt, abfiltriert und der Filterkuchen mit VE-Wasser nachgewaschen. Der Filterkuchen wurde bei 100 C vorgetrocknet und bei 750°C 30 min kalziniert. Es wurde ein sehr brillantes, farbstarkes, bronzefarbenes Mehrschichtperlglanzpigment erhalten.

**Vergleichsbeispiel 8: Synthetischer Glimmer/ Fe$_2$O$_3$/ SiO$_2$/ Fe$_2$O$_3$**

200 g synthetischer Glimmer aus Vergleichsbeispiel 2 wurden in 2000 ml VE-Wasser suspendiert und unter turbulentem Rühren auf 80°C erhitzt. Der pH-Wert der Suspension wurde mit verdünnter HCl auf pH 3,0 eingestellt und sodann eine Lösung von 37,5 ml FeCl$_3$ (280 g Fe$_2$O$_3$/l VE-Wasser) in die Suspension hinzudosiert. Der pH-Wert wurde dabei durch Gegensteuern mit 10 Gew.-%iger NaOH-Lösung konstant auf pH 3,0 gehalten. Um die Fällung zu vervollständigen wurde die Suspension noch weitere 15 min gerührt. Danach wurde der pH-Wert mit 5 Gew.-%iger NaOH-Lösung auf 7,5 angehoben und 15 min gerührt. Eine Wasserglaslösung (153 g Wasserglaslösung, 20 Gew.-% SiO$_2$, gemischt mit 207 g VE-Wasser) wurde sodann langsam in die Suspension eingeleitet und der pH-Wert konstant bei pH 7,5 gehalten. Danach wurde noch 20 min nachgerührt und der pH-Wert wieder auf 3,0 gesenkt und sodann eine Lösung von 120 ml FeCl$_3$ (280 g Fe$_2$O$_3$/ l VE-Wasser) in die Suspension hinzudosiert. Der pH-Wert wurde dabei durch Gegensteuern mit 10 Gew.-%iger NaOH-Lösung konstant auf pH 3,0 gehalten. Danach wurde noch 15 min nachgerührt, abfiltriert und der Filterkuchen mit VE-Wasser nachgewaschen. Der Filterkuchen wurde bei 100 C vorgetrocknet und bei 750°C 30 min kalziniert. Es wurde ein schwach glänzendes, bronzefarbenes Mehrschichtperlglanzpigment erhalten.

**Vergleichsbeispiel 9: Synthetischer Glimmer/ Fe$_2$O$_3$/ SiO$_2$/ TiO$_2$(rutil)**

200 g synthetischer Glimmer aus Vergleichsbeispiel 2 wurden in 2000 ml VE-Wasser suspendiert und unter turbulentem Rühren auf 80°C erhitzt. Der pH-Wert der Suspension wurde mit verdünnter HCl auf pH 3,0 eingestellt und sodann eine Lösung von 37,5 ml FeCl$_3$ (280 g Fe$_2$O$_3$/l VE-Wasser) in die Suspension hinzudosiert. Der pH-Wert wurde dabei durch Gegensteuern mit 10 Gew.-%-iger NaOH-Lösung konstant auf pH 3,0 gehalten. Um die Fällung zu vervollständigen wurde die Suspension noch weitere 15 min gerührt. Danach wurde der pH-Wert mit 5 Gew.-%-iger NaOH-Lösung auf 7,5 angehoben und 15 min gerührt. Eine Wasserglaslösung (153 g Wasserglaslösung, 20 Gew.-% SiO$_2$,

gemischt mit 207 g VE-Wasser) wurde sodann langsam in die Suspension eingeleitet und der pH-Wert konstant bei pH 7,5 gehalten. Danach wurde noch 20 min nachgerührt und der pH-Wert wieder auf 1,9 gesenkt. Dann schied man eine Schicht "SnO$_2$" auf der SiO$_2$-Oberfläche ab. Diese Schicht wurde durch Zugabe von einer Lösung, bestehend aus 5 g SnCl$_4$ x 5 H$_2$O (in 10 ml konz. HCl plus 50 ml VE-Wasser), unter gleichzeitiger Zudosierung einer 10 Gew.-%-igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 1,6 heruntergesetzt und sodann eine Lösung von 300 ml TiCl$_4$ (200 g TiO$_2$/l VE-Wasser) in die Suspension hinzudosiert. Der pH-Wert wurde dabei durch Gegensteuern mit 10 Gew.-%iger NaOH-Lösung konstant auf pH 1,6 gehalten. Danach wurde noch 15 min nachgerührt, abfiltriert und der Filterkuchen mit VE-Wasser nachgewaschen. Der Filterkuchen wurde bei 100 C vorgetrocknet und bei 750°C 30 min kalziniert. Es wurde ein schwach glänzendes, bronzefarbenes Mehrschichtperlglanzpigment erhalten.

## II Physikalische Charakterisierung

### IIa Winkelabhängige Farbmessungen

Zur Messung der Chromawerte wurden die Mehrschichtperlglanzpigmente mit einer Pigmentierungshöhe von 6 Gew.-% (bezogen auf das Gesamtgewichtes des Nasstacks) in einen konventionellen Nitrocelluloselack (Dr. Renger Erco Bronzemischlack 2615e; Fa. Morton) eingerührt. Dabei wurden die Mehrschichtperlglanzpigmente vorgelegt und anschließend mit einem Pinsel in den Lack dispergiert.

Der fertige Lack wurde auf einem Rakelabzugsgerät (RK Print Coat Instr. LTd. Citenco Abziehgerät Modell K 101) mit einer Nassfilmdicke je nach D$_{50}$-Wert des Mehrschichtperlglanzpigments gemäß Tabelle 2 auf Byk-Gardner Schwarz-Weiß-Rakelkarten (Byko-Chart 2853) appliziert und nachfolgend bei Raumtemperatur getrocknet.

Mit dem Mehrwinkelfarbmessgerät, Byk Mac (Fa. Byk Gardener), wurden bei einem konstanten Einfallswinkel von 45° (gemäß Herstellerangaben) bei verschiedenen Beobachtungswinkeln relativ zum Glanzwinkel die L*- und C*-Werte bestimmt. Insbesondere waren die Beobachtungswinkel relativ nahe am Glanzwinkel bei 15° sowie -15° relevant. Als relevanter Chromawert der erfindungsgemäßen Mehrschichtperlglanzpigmente wurde der C*$_{15}$-Wert herangezogen, welcher bei einem 15° vom Glanz entfernten Winkel gemessen wurde.

Stark reflektierende Proben (Idealfall Spiegel) reflektierten nahezu das gesamte eintreffende Licht im sogenannten Glanzwinkel. Hier erschien der Farbeindruck der Interferenzfarbe am stärksten. Je weiter man sich bei der Messung vom Glanzwinkel entfernte, desto weniger Licht und somit Interferenzeffekt konnte gemessen werden.

### IIb Glanzmessungen

Der Glanz ist ein Maß für die gerichtete Reflexion und lässt sich mittels eines Micro-Tri-Gloss-Gerätes charakterisieren. Stärker streuende Proben weisen mithin einen niedrigen Glanz auf.

Es wurden die Nitrolackapplikationen aus IIa mit Hilfe eines Micro-Tri-Gloss Glanzmessgerätes der Fa. Byk Gardner bei einem Messwinkel von 20° für hochglänzende Proben und bei 60° für mittelglänzende Proben auf schwarzem Hintergrund vermessen. Lagen die Glanzwerte bei 60° über 70 Glanzeinheiten, so werden die Proben bei 20° gemessen (Byk-Gardner Katalog 2007/ 2008, S. 14).

### IIc Partikelgrößenbestimmung:

Die Größenverteilungskurve wurde mit einem Gerät der Fa. Malvern (Gerät: MALVERN Mastersizer 2000) gemäß Herstellerangaben bestimmt. Hierzu wurden ca. 0,1g des entsprechenden Pigmentes als wässrige Suspension, ohne Zusatz von Dispergierhilfsmitteln, unter ständigem Rühren mittels einer Pasteurpipette in die Probenvorbereitungszelle des Messgerätes gegeben und mehrmals vermessen. Aus den einzelnen Messergebnissen wurden die resultierenden Mittelwerte gebildet. Die Auswertung der Streulichtsignale erfolgte dabei nach der Mie-Theorie, welche auch Brechungs- und Absorptionsverhalten der Partikel beinhaltet (Abbildung 1).

Unter der mittleren Größe D$_{50}$ wird im Rahmen dieser Erfindung der D$_{50}$-Wert der Summendurchgangskurve der Volumengemittelten Größenverteilungsfunktion, wie sie durch Laserbeugungsmethoden erhalten werden, verstan-den. Der D$_{50}$-Wert gibt an, dass 50 % der Pigmente einen Durchmesser aufweisen, der gleich oder kleiner als der angegebene Wert, beispielsweise 20 $\mu$m, ist.

Entsprechend gibt der D$_{90}$-Wert an, dass 90% der Pigmente einen Durchmesser aufweisen, der gleich oder kleiner des jeweiligen Wertes ist.

Weiterhin gibt der D$_{10}$-Wert an, dass 10% der Pigmente einen Durchmesser aufweisen, der gleich oder kleiner des jeweiligen Wertes ist.

Der Span $\Delta$D, definiert als $\Delta D = (D_{90} - D_{10})/D_{50}$, gibt die Verteilungsbreite wieder.

**III. Ergebnisse**

Tabelle 3: Charakterisierung der Effektpigmente

| Effektpigment | Aufbau | Glanz, 20° | $C^*_{15}$ | Span |
|---|---|---|---|---|
| Vergleichsbeispiel 3 | Glasflake/$Fe_2O_3$ | 62,9 | 4,1 | 1,1 |
| Vergleichsbeispiel 4 | Glasflake/$Fe_2O_3$ | 46,7 | 4,0 | 2,0 |
| Beispiel 3 | Glasflake/$Fe_2O_3$/$SiO_2$/$Fe_2O_3$ | 79,4 | 31,5 | 1,1 |
| Beispiel 4 | Glasflake/$Fe_2O_3$/$SiO_2$/$TiO_2$ | 82,5 | 27,7 | 1,1 |
| Vergleichsbeispiel 6 | Glasflake/$Fe_2O_3$/$SiO_2$/$Fe_2O_3$ | 49,4 | 22,7 | 2,0 |
| Vergleichsbeispiel 7 | Glasflake/$Fe_2O_3$/$SiO_2$/$TiO_2$ | 50,9 | 20,4 | 2,0 |

Anhand der Daten in Tabelle 3 kann man eindeutig erkennen, dass die erfindungsgemäßen Beispiele 3 und 4 mit dem Schichtaufbau Glasflake/$Fe_2O_3$/$SiO_2$/$Fe_2O_3$ bzw. Glasflake/$Fe_2O_3$/$SiO_2$/$TiO_2$ und geringem Span mit 16,5 Einheiten bzw. 19,6 Einheiten einen starken Glanzgewinn im Vergleich zum Ausgangsmaterial (Vergleichsbeispiel 3) aufwies. Für die Vergleichsbeispiele 6 und 7 mit großem Span fand man lediglich einen leicht erhöhten Glanzwert im Vergleich zum Ausgangsmaterial Vergleichsbeispiel 4. Auch das Chroma der erfindungsgemäßen Beispiele 3 und 4 zeigt sich gegenüber den Vergleichsbeispielen 6 und 7 mit breitem span signifikant erhöht.

Tabelle 4: Charakterisierung der Effektpigmente

| Effektpigment | Aufbau | Glanz, 60° | $C^*_{15}$ | Span |
|---|---|---|---|---|
| Beispiel 5 | synth. Glimmer/ $Fe_2O_3$/ $SiO_2$/ $Fe_2O_3$ | 59,2 | 33,6 | 1,2 |
| Beispiel 6 | synth. Glimmer/ $Fe_2O_3$/ $SiO_2$/ $TiO_2$ | 57,2 | 25,3 | 1,2 |
| Vergleichsbeispiel 5 | synth. Glimmer/ $Fe_2O_3$ | 26,4 | 22,3 | 1,2 |
| Vergleichsbeispiel 8 | synth. Glimmer/ $Fe_2O_3$/ $SiO_2$/ $Fe_2O_3$ | 29,3 | 26,5 | 3,7 |
| Vergleichsbeispiel 9 | synth. Glimmer/ $Fe_2O_3$/ $SiO_2$/ $TiO_2$ | 23,9 | 18,8 | 3,7 |

Auch bei den Glimmer-basierenden erfindungsgemäßen Beispielen 5 und 6 ist laut Tabelle 4 ein extremer Glanzsteigerungseffekt besonders zu den Vergleichsbeispielen 8 und 9 (gleicher Schichtaufbau) mit breitem Span zu beobachten. Weiterhin zeigt sich eine zusätzliche Glanzsteigerung aufgrund der Multischichttechnologie im Vergleich Beispiel 5 und 6 zu Vergleichsbeispiel 5.

**IV. Anwendungstechnische Beispiele**

In den nachfolgenden kosmetischen Anwendungsbeispielen wurden die erfindungsgemäßen Mehrschichtperlglanzpigmente verwendet, die nach einem der vorstehenden Beispiele hergestellt wurden.

**Beispiel 7:** Nagellack

| INCI Name | Produktname | Gew-% | Lieferant |
|---|---|---|---|
| *Phase A* | | *100.00* | |
| **Mehrschichtperlglanz pigment** | **Mehrschichtperlglanzpigment** | **2.00** | |

(fortgesetzt)

| INCI Name | Produktname | Gew-% | Lieferant |
|---|---|---|---|
| *Phase B* | | | |
| Butylacetat (and) Ethylacetat (and) Nitrocellulose (and) Isopropyl Alcohol | International Lacquers Nailpolish & Care Base 359 | 98.00 | www.internationallacqu ers.lu |

Das Mehrschichtperlglanzpigment kann in einem Bereich von 0,1 -10,0 Gew.-% eingesetzt werden. Der Ausgleich kann mit International Lacquers Nailpolish erfolgen.

Phase A und Phase B wurden gemischt und anschließend in ein angemessenes Behältnis abgefüllt.

**Beispiel 8:** Creme Lidschatten

| INCI Name | Produktname | Gew-% | Lieferant |
|---|---|---|---|
| *Phase A* | | 100.00 | |
| Castor Oil | Castor Oil | 28.70 | www.riedeldehaen. com |
| Octyl Palmitate | Liponate EHP | 6.00 | www.lipochemicals. com |
| Cocos Nucifera (Coconut) Oil | Lipovol C-76 | 7.00 | www.lipochemicals. com |
| Bees Wax | Ewacera 12 | 6.00 | www.wagnerlanolin .com |
| Isopropyl Lanolate | Ewalan IP | 5.00 | www.wagnerlanolin .com |
| Persea Gratissima (Avocado) Oil and Hydrogenated Avocado Oil | Avocado Butter | 7.00 | www.impag.de |
| Magnesium Stearate | Magnesium Stearate | 3.00 | www.sigmaaldrich.c om |
| Bis-Hydroxyethoxypropyl Dimethicone | Dow Corning 5562 Carbinol Fluid | 7.00 | www.dowcoming.c om |
| Dimethicone/ Vinyl Dimethicone Crosspolymer and Silica | Dow Corning 9701 Cosmetic Powder | 5.00 | www.dowcorning.c om |
| Phenoxyethanol (and) Methylparaben (and) Ethylparaben (and) Butylparaben | Uniphen P-23 | 0.30 | www.induchem.co m |
| *Phase B* | | | |
| **Mehrschichtperlglanzpigment** | **Mehrschichtperlglanzpigment** | **25.00** | |

Das Mehrschichtperlglanzpigment kann in einem Bereich von 5,0 - 30,0 Gew.-% eingesetzt werden. Der Ausgleich kann mit Castor Oil erfolgen.

Phase A wurde gemischt und auf 85°C erhitzt, Inhaltsstoffe von Phase B wurden ebenfalls zusammengemischt und anschließend unter Rühren zu Phase A hinzugegeben. Nach Abfüllen in ein entsprechendes Behältnis wurde die Mischung auf Raumtemperatur abgekühlt.

**Beispiel 9:** Foundation

| INCI Name | Produktname | Gew-% | Lieferant |
|---|---|---|---|
| *Phase* A | | 100.00 | |
| Hydrogenated Polydecene | Ritadecene 20 | 9.00 | www.ritacorp.com |

(fortgesetzt)

| INCI Name | Produktname | Gew-% | Lieferant |
|---|---|---|---|
| *Phase* A | | 100.00 | |
| Caprylic/ Capric Triglyceride | Liponate GC-K | 5.00 | www.lipochemicals.c om |
| Prunus Amygdalus Dulcis (Sweet Almond) Oil | Sweet Almond Oil | 4.00 | www.jandekker.com |
| Caprylyl Trimethicone | SilCare Silicone 31M50 | 4.00 | www.clariant.com |
| Caprylyl Methicone | SilCare Silicone **41M15** | 3.00 | www.clariant.com |
| Steareth-2 | Volpo S2 | 1.60 | www.croda.com |
| Steareth-20 | Sympatens AS/200 G | 2.40 | www.kolb.ch |
| *Phase B* | | | |
| Talc | Talc Powder | 4.50 | www.vwr.com |
| Mica (and) Iron Oxides | Prestige Soft Beige | 4.00 | |
| Mica (and) Titanium Dioxide | Prestige Soft Silver | 1.00 | www.eckart.net |
| **Mehrschichtperlglanzpigment** | **Mehrschichtperlglanzpigment** | **2.00** | |
| *Phase C* | | | |
| Glycerin | Pricerine 9090 | 5.00 | www.brenntag.com |
| Aqua | Wasser | 53.70 | |
| Ammonium Acryloyldimehtyltauratel VP Copolymer | Aristoflex AVC | 0.40 | www.simon-und-werner.com |
| *Phase D* | | | |
| Propylene Glycol (and) Diazolidinyl Urea (and) Methylparaben (and) Propylparaben | Nipaguard PDU | 0.40 | www.simon-und-werner.com |

Das Mehrschichtperlglanzpigment kann in einem Bereich von 0,1 - 8,0 Gew.-% eingesetzt werden. Der Ausgleich kann mit Wasser erfolgen.

Phase A und Phase B wurden separat eingewogen. Phase A wurde unter Rühren auf 70°C erhitzt und Phase B unter Rühren hinzugefügt. Phase C wurde gut gemischt bis Aristoflex aufgelöst war und anschließend ebenfalls auf 70°C erhitzt. Phase C wurde zu Phase AB hinzugefügt und nach Abkühlen auf 40°C wurde Phase D hinzugefügt.

**Beispiel 10:** Gepresster Lidschatten

| INCI Name | Produktname | Gew-% | Lieferant |
|---|---|---|---|
| *Phase A* | | 100.00 | |
| Mica | Silk Mica | 17.00 | www.vwr.com |
| Boron Nitride | Softouch CCS 102 | 2.50 | www.advceramicsc os.com |
| Zinc Stearate | Kemilub EZ-V | 7.00 | www.undesa.com |
| Talc | Talc Powder | 38.50 | www.riedeldehaen. com |
| **Mehrschichtperlglanzpigment** | **Mehrschichtperlglanzpigment** | **25.00** | |

(fortgesetzt)

| Phase B | | | |
|---|---|---|---|
| Dimethicone | Dow Corning 200 Fluid 5 cst. | 5.00 | www.dowcorning.c om |
| Cyclomethicone (and) Dimethicone Crosspolymer | Dow Corning 9040 Elastomer | 5.00 | www.dowcoming.c om |

Das Mehrschichtperlglanzpigment kann in einem Bereich von 5,0 - 40,0 Gew.-% eingesetzt werden. Der Ausgleich kann mit Talc erfolgen.

Phase A wurde für 30s bei 2500 rpm in einem Hochgeschwindigkeitsmixer gemischt. Anschließend wurde Phase B zugegeben und die Mischung für 60s bei 3000 rpm im gleichen Mixer gemischt. Zuletzt wurde die Pulvermischung mittels einer Lidschattenpresse bei 150 bar für 30s in Form gepresst.

**Beispiel 11:** Haar Mascara

| INCI Name | Produktname | Gew-% | Lieferant |
|---|---|---|---|
| Phase A | | 100.00 | |
| Polyquaternium-16 | Luviquat FC 905 (Luviquat Exellence) | 2.70 | www.basf.com |
| Propylene Glycol | 1,2-Propanediol | 1.80 | www.vwr.com |
| Methylparaben | Methyl-4-hydroxybenzoate | 0.20 | www.sigmaaldrich.com |
| Aqua | Wasser | 64.95 | |
| Phase B | | | |
| Cetearyl Alcohol | Lanette O | 5.00 | www.cognis.com |
| Dimethicone | Dow Corning 200 Fluid/ 350 cst | 1.00 | www.dowcorning.com |
| Ceteareth-25 | Cremophor A 25 | 2.00 | www.basf.com |
| Propylparaben | Propyl-4-hydroxybenzoate | 0.10 | www.sigmaaldrich.com |
| Phase C | | | |
| Hydroxypropylcellulose | Klucel G | 0.50 | www.herc.com |
| Magnesium Aluminium Silicate | Veegum HV | 0.50 | www.rtvanderbilt.com |
| Aqua | Wasser | 19.00 | |
| Phase D | | | |
| **Mehrschichtperlglanz pigment** | **Mehrschichtperlglanzpigment** | **2.00** | |
| Phenoxyethanol (and) Methylparaben (and) Butylparaben (and) Ethylparaben (and) Propylparaben (and) Isobutylparaben | Phenonip | 0.20 | www.clariant.com |
| Fragrance | Blue Shadow ÖKO | 0.05 | www.bell-europe.com |

Das Mehrschichtperlglanzpigment kann in einem Bereich von 0,5 - 5,0 Gew.-% eingesetzt werden. Der Ausgleich kann mit Wasser von Phase A erfolgen.

Phase A und Phase B wurden getrennt auf 80 ° C erhitzt; danach wurde Phase B langsam zu Phase A hinzugefügt. In einem separaten Gefäß wurden Klucel und Veegum dem Wasser von Phase C hinzugegeben. Anschließend wurde Phase AB auf 40°C abgekühlt und während dem Abkühlen Phase C und D unter rühren hinzugemischt.

**Beispiel 12:** Haargel

| INCI Name | Produktname | Gew-% | Lieferant |
|---|---|---|---|
| *Phase A* | | 100.00 | |
| **Mehrschichtperlglanzpigment** | **Mehrschichtperlglanzpigment** | **0.10** | |
| Ammonium Acryloyldimehtyltaurate/ VP Copolymer | Aristoflex AVC | 1.40 | www.clariant.com |
| Citric Acid | Citric Acid | 0.10 | www.vwr.com |
| Aqua | Wasser | 55.10 | |
| *Phase B* | | | |
| PVP | Luviskol K 30 Powder | 1.50 | www.basf.com |
| Propylene Glycol, Diazolidinyl, Urea, Methylparaben, Propylparaben | Germaben II | 0.20 | www.ispcorp.com |
| Triethanolamine | Triethanolamine | 1.20 | www.vwr.com |
| Water | Aqua | 40.40 | |

Das Mehrschichtperlglanzpigment kann in einem Bereich von 0,01 - 0,5 Gew.-% eingesetzt werden. Der Ausgleich kann mit Wasser erfolgen.

Das Pigment wurde mit dem Wasser von Phase A verrührt, Aristoflex AVP und Citric Acid wurde unter Rühren hinzugegeben und bei einer Geschwindigkeit von 800 rpm für 15 Minuten gemischt. Die Inhaltsstoffe von Phase B wurden aufgelöst bis eine homogene Lösung entstand, anschließend wurde Phase B zu Phase A gegeben und gemischt.

**Patentansprüche**

**1.** Mehrschichtperlglanzpigmente, umfassend mit optisch wirksamer Beschichtung versehene plättchenförmige transparente Substrate,
**dadurch gekennzeichnet,**
**dass** die optisch wirksame Beschichtung wenigstens

(a) eine absorbierende hochbrechende Schicht A mit einem Brechungsindex n $\geq$ 1,8,
(b) eine niedrigbrechende Schicht B mit einem Brechungsindex n < 1,8,
(c) eine hochbrechende Schicht C mit einem Brechungsindex n $\geq$ 1,8 sowie
(d) optional wenigstens eine äußere Schutzschicht D

umfasst und
**dass** die Mehrschichtperlglanzpigmente eine Summenhäufigkeitsverteilung einer volumengemittelten Größenverteilungsfunktion mit den Kennzahlen $D_{10}$, $D_{50}$, $D_{90}$ und einem Span $\Delta D$ in einem Bereich von 0,7 - 1,4 aufweisen, wobei der Span $\Delta D$ gemäß Formel (I)

$$\Delta D = (D_{90} - D_{10})/ D_{50} \qquad (I)$$

berechnet ist.

**2.** Mehrschichtperlglanzpigmente nach Anspruch 1,
**dadurch gekennzeichnet,**
**dass** die Mehrschichtperlglanzpigmente keine silberne Interferenzfarbe aufweisen.

**3.** Mehrschichtperlglanzpigmente nach einem der vorstehenden Ansprüche,

**dadurch gekennzeichnet,**
**dass** die Mehrschichtperlglanzpigmente einen Span ∆D in einem Bereich von 0,7 bis 1,3 aufweisen.

4. Mehrschichtperlglanzpigmente nach einem der vorstehenden Ansprüche,
   **dadurch gekennzeichnet,**
   **dass** die optische Schichtdicke der Schicht A in einem Bereich von 30 bis 900 nm liegt.

5. Mehrschichtperlglanzpigmente nach einem der vorstehenden Ansprüche,
   **dadurch gekennzeichnet,**
   **dass** die optische Schichtdicke der Schicht B in einem Bereich von 30 bis ≤ 500 nm liegt.

6. Mehrschichtperlglanzpigmente nach einem der vorstehenden Ansprüche,
   **dadurch gekennzeichnet,**
   **dass** die optische Schichtdicke der Schicht B in einem Bereich von 30 bis 150 nm liegt.

7. Mehrschichtperlglanzpigmente nach einem der vorstehenden Ansprüche,
   **dadurch gekennzeichnet,**
   **dass** die optische Schichtdicke der Schicht C in einem Bereich von 30 bis 900 nm liegt.

8. Mehrschichtperlglanzpigmente nach einem der vorstehenden Ansprüche,
   **dadurch gekennzeichnet,**
   **dass** die Schichten A und C Titanoxid, vorzugsweise Titandioxid, umfassen.

9. Mehrschichtperlglanzpigmente nach einem der vorstehenden Ansprüche,
   **dadurch gekennzeichnet,**
   **dass** die Schicht B Siliziumoxid, vorzugsweise Siliziumdioxid, umfasst.

10. Mehrschichtperlglanzpigmente nach einem der vorstehenden Ansprüche,
    **dadurch gekennzeichnet,**
    **dass** die plättchenförmigen Substrate aus der Gruppe, bestehend aus natürlichem Glimmer, synthetischem Glimmer, Glasflakes, $SiO_2$-Plättchen, $Al_2O_3$-Plättchen und deren Gemische, ausgewählt ist.

11. Verfahren zum Herstellen der Mehrschichtperlglanzpigmente nach einem der vorstehenden Ansprüche,
    **dadurch gekennzeichnet,**
    **dass** das Verfahren folgende Schritte umfasst:

    (i) Größenklassieren der zu beschichtenden plättchenförmigen transparenten Substrate, so dass die zu beschichtenden plättchenförmigen transparenten Substrate eine volumengemittelte Größenverteilungsfunktion mit den Kennzahlen $D_{10}$, $D_{50}$, $D_{90}$ und einem Span ∆D in einem Bereich von 0,7 - 1,4 aufweisen, wobei der Span ∆D gemäß Formel (I) definiert ist,
    (ii) Aufbringen wenigstens der Schichten A bis C auf die plättchenförmigen transparenten Substrate sowie optional wenigstens einer Schicht D,
    oder
    (iii) Aufbringen wenigstens der Schichten A bis C auf die plättchenförmigen transparenten Substrate sowie optional wenigstens einer Schicht D,
    (iv) Größenklassieren der zu beschichtenden plättchenförmigen transparenten Substrate, so dass die zu beschichtenden plättchenförmigen Substrate eine volumengemittelte Größenverteilungsfunktion mit den Kennzahlen $D_{10}$, $D_{50}$, $D_{90}$ und einem Span ∆D in einem Bereich von 0,7 -1,4 aufweisen, wobei der Span ∆D gemäß Formel (I) definiert ist.

12. Verwendung der Mehrschichtperlglanzpigmente nach einem der Ansprüche 1 bis 10 in kosmetischen Formulierungen, Kunststoffen, Folien, Textilien, keramischen Materialien, Gläsern und Beschichtungszusammensetzungen wie Farben, Druckfarben, Lacken oder Pulverlacken.

13. Gegenstand,
    **dadurch gekennzeichnet,**
    **dass** der Gegenstand die Mehrschichtperlglanzpigmente nach einem der Ansprüche 1 bis 10 aufweist oder enthält.

**14.** Zubereitung,
**dadurch gekennzeichnet,**
**dass** die Zubereitung die Mehrschichtperlglanzpigmente nach einem der Ansprüche 1 bis 10 aufweist oder enthält.


**Claims**

**1.** Multi-layered pearlescent pigments comprising platelet-shaped, transparent substrates provided with an optically active coating,
**characterised in that**
the optically active coating comprises at least

a) an absorbing, high-index layer A with a refractive index $n \geq 1.8$,
b) a low-index layer B with a refractive index $n < 1.8$,
c) a high-index layer C with a refractive index $n \geq 1.8$,
and
d) optionally at least one outer protective layer D,

and the multi-layered pearlescent pigments have a cumulative frequency distribution of a volume-averaged size distribution function with the indices $D^{10}$, $D^{50}$ and $D^{90}$ and a span $\Delta D$ in a range of 0.7 - 1,4, the span $\Delta D$ being calculated in accordance with equation (I)

$$\Delta D = (D^{90}\text{-}D^{10})/D^{50} \qquad (I).$$

**2.** Multi-layered pearlescent pigments as claimed in claim 1,
**characterised in that**
the multi-layered pearlescent pigments do not contain any silver interference colour.

**3.** Multi-layered pearlescent pigments as claimed in one of the preceding claims,
**characterised in that**
the multi-layered pearlescent pigments have a span $\Delta D$ in a range of 0.7 to 1.3.

**4.** Multi-layered pearlescent pigments as claimed in one of the preceding claims,
**characterised in that**
the optical layer thickness of layer A lies in a range of 30 to 900 nm.

**5.** Multi-layered pearlescent pigments as claimed in one of the preceding claims,
**characterised in that**
the optical layer thickness of layer B lies in a range of 30 to $\leq$ 500 nm.

**6.** Multi-layered pearlescent pigments as claimed in one of the preceding claims,
**characterised in that**
the optical layer thickness of layer B lies in a range of 30 to 500 nm.

**7.** Multi-layered pearlescent pigments as claimed in one of the preceding claims,
**characterised in that**
the optical layer thickness of layer C lies in a range of 30 nm to 900 nm.

**8.** Multi-layered pearlescent pigments as claimed in one of the preceding claims,
**characterised in that**
layers A and C comprise titanium oxide, preferably titanium dioxide.

**9.** Multi-layered pearlescent pigments as claimed in one of the preceding claims,
**characterised in that**
layer B comprises silicon oxide, preferably silicon dioxide.

**10.** Multi-layered pearlescent pigments as claimed in one of the preceding claims, **characterised in that** the platelet-shaped, transparent substrates of the multi-layered pearlescent pigments are selected from the group comprising natural mica, synthetic mica, glass flakes, $SiO_2$ platelets, $Al_2O_3$ platelets and mixtures thereof.

**11.** Method of producing multi-layered pearlescent pigments as claimed in one of the preceding claims, **characterised in that** the method comprises the following steps:

(i) classifying the size of the platelet-shaped, transparent substrates to be coated so that the platelet-shaped, transparent substrates to be coated have a volume-averaged size distribution function with the indices $D^{10}$, $D^{50}$, $D^{90}$ and a span $\Delta D$ in a range of 0.7 - 1.4, the span $\Delta D$ being defined by equation (I),
(ii) applying at least layers A to C to the platelet-shaped, transparent substrates and optionally at least a layer D or
(iii) applying at least layers A to C to the platelet-shaped, transparent substrates and optionally at least a layer D,
(iv) classifying the size of the platelet-shaped, transparent substrates to be coated so that the platelet-shaped, transparent substrates to be coated have a volume-averaged size distribution function with the indices $D^{10}$, $D^{50}$, $D^{90}$ and a span $\Delta D$ in a range of 0.7 - 1.4, the span $\Delta D$ being defined by equation (I).

**12.** Use of the multi-layered pearlescent pigments as claimed in one of claims 1 to 10 in cosmetic formulations, plastics, foils, textiles, ceramic materials, glasses and coating compositions such as paints, printing inks, varnishes or powder coatings.

**13.** Object **characterised in that** the object has or contains the multi-layered pearlescent pigments as claimed in one of claims 1 to 10.

**14.** Preparation **characterised in that** the preparation has or contains the multi-layered pearlescent pigments as claimed in one of claims 1 to 10.

**Revendications**

**1.** Pigments nacrés multicouches, comprenant des substrats transparents lamellaires pourvus d'un revêtement optiquement actif, **caractérisés en ce que** le revêtement optiquement actif comprend au moins

(a) une couche absorbante A à grand indice de réfraction, ayant un indice de réfraction n ≥ 1,8,
(b) une couche B à faible indice de réfraction, ayant un indice de réfraction n < 1,8,
(c) une couche C à grand indice de réfraction, ayant un indice de réfraction n ≥ 1,8, ainsi que
(d) en option, au moins une couche de protection extérieure D, et

**en ce que** les pigments nacrés multicouches présentent une distribution de fréquences cumulées d'une fonction de distribution granulométrique moyennée en volume, présentant les fractions $D_{10}$, $D_{50}$, $D_{90}$, et un span $\Delta D$ compris dans la plage de 0,7 à 1,4, le span $\Delta D$ étant calculé selon la formule (I)

$$\Delta D = (D_{90} - D_{10})/D_{50} \qquad (I)$$

**2.** Pigments nacrés multicouches selon la revendication 1, **caractérisés en ce que** les pigments nacrés multicouches ne comportent pas de couleurs d'interférence argent.

**3.** Pigments nacrés multicouches selon l'une des revendications précédentes, **caractérisés en ce que** les pigments nacrés multicouches présentent un span $\Delta D$ compris dans la plage de 0,7 à 1,3.

**4.** Pigments nacrés multicouches selon l'une des revendications précédentes, **caractérisés en ce que** l'épaisseur de couche optique de la couche A est comprise dans la plage de 30 à 900 nm.

**5.** Pigments nacrés multicouches selon l'une des revendications précédentes, **caractérisés en ce que** l'épaisseur de couche optique de la couche B est comprise dans la plage de 30 à $\leq$ 500 nm.

**6.** Pigments nacrés multicouches selon l'une des revendications précédentes, **caractérisés en ce que** l'épaisseur de couche optique de la couche B est comprise dans la plage de 30 à 150 nm.

**7.** Pigments nacrés multicouches selon l'une des revendications précédentes, **caractérisés en ce que** l'épaisseur de couche optique de la couche C est comprise dans la plage de 30 à 900 nm.

**8.** Pigments nacrés multicouches selon l'une des revendications précédentes, **caractérisés en ce que** les couches A et C comprennent un oxyde de titane, de préférence du dioxyde de titane.

**9.** Pigments nacrés multicouches selon l'une des revendications précédentes, **caractérisés en ce que** la couche B comprend un oxyde de silicium, de préférence du dioxyde de silicium.

**10.** Pigments nacrés multicouches selon l'une des revendications précédentes, **caractérisés en ce que** les substrats lamellaires sont choisis dans le groupe consistant en le mica naturel, le mica synthétique, les écailles de verre, les lamelles de $SiO_2$, les lamelles d'$Al_2O_3$ et les mélanges de ceux-ci.

**11.** Procédé de fabrication des pigments nacrés multicouches selon l'une des revendications précédentes, **caractérisé en ce que** le procédé comprend les étapes suivantes :

(i) classification granulométrique des substrats transparents lamellaires devant être revêtus, de telle sorte que les substrats transparents lamellaires devant être revêtus présentent une fonction de distribution granulométrique moyennée en volume présentant les fractions $D_{10}$, $D_{50}$, $D_{90}$ et un span $\Delta D$ compris dans la plage de 0,7 à 1,4, le span $\Delta D$ étant défini selon la formule (I),
(ii) application d'au moins les couches A et C sur les substrats transparents lamellaires, ainsi qu'en option au moins d'une couche D, ou
(iii) application d'au moins les couches A et C sur les substrats transparents lamellaires, ainsi qu'en option au moins d'une couche D,
(iv) classification granulométrique des substrats transparents lamellaires devant être revêtus, de telle sorte que les substrats lamellaires devant être revêtus présentent une fonction de distribution granulométrique moyennée en volume présentant les fractions $D_{10}$, $D_{50}$, $D_{90}$ et un span $\Delta D$ compris dans la plage de 0,7 à 1,4, le span $\Delta D$ étant défini selon la formule (I).

**12.** Utilisation des pigments nacrés multicouches selon l'une des revendications 1 à 10 dans des formulations cosmétiques, des plastiques, des feuilles, des textiles, des matériaux céramiques, des verres et des compositions de revêtement, ainsi que des encres, des encres d'imprimerie, des vernis et des vernis en poudre.

**13.** Objet, **caractérisé en ce que** l'objet présente ou contient les pigments nacrés multicouches selon l'une des revendications 1 à 10.

**14.** Préparation, **caractérisée en ce que** la préparation présente ou contient les pigments nacrés multicouches selon l'une des revendications 1 à 10.

Abbildung 1: Beeinflussung der Laserbeugung durch Partikeleigenschaften

d = Partikeldurchmesser

Partikeleigenschaften:

d: Durchmesser

1:Beugung

2: Brechung

3 Reflexion

4: Absorption

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4141069 A1
- DE 4319669 A1
- DE 19614637 A1
- DE 19802234 A1
- DE 19808657 A1
- DE 19822046 A1
- DE 19907313 A1
- DE 19941253 A1
- DE 19953655 A1
- DE 10221497 A1
- WO 2004055119 A1
- WO 2002090448 A2
- EP 0753545 B2
- WO 2004067645 A2
- EP 0948572 B1
- WO 2006110359 A2
- US 20060042509 A1
- WO 2009103322 A1
- WO 03006558 A2
- EP 0289240 A1
- WO 2004056716 A1
- WO 2005063637 A1
- EP 1980594 B1
- WO 2006021386 A1